# FASCICULE DE BREVET EUROPEEN

(11) **EP 2 734 611 B1**
(45) Date de publication et mention de la délivrance du brevet: **15.04.2015**
(21) Numéro de dépôt: 12743510.5
(22) Date de dépôt: 12.07.2012
(51) Int. Cl.: A61J 1/20, A61J 1/14, B65D 25/00, C12M 3/00, A61M 39/18

(54) **PERFECTIONNEMENT AU RACCORDEMENT D'UN ACCESSOIRE À UN RÉCIPIENT**
VERBESSERUNG DER VERBINDUNG EINES ZUBEHÖRTEILS MIT EINEM BEHÄLTER
IMPROVEMENT TO THE CONNECTION OF AN ACCESSORY TO A RECEPTACLE

(30) Priorité: 19.07.2011 FR 1156540
(43) Date de publication de la demande: 28.05.2014
(73) Titulaire: SARTORIUS STEDIM FMT SAS, 13400 Aubagne (FR)
(72) Inventeur: BERNARD, Frédéric, F-83740 La Cadiere d'Azur (FR); CHAUSSIN, Sébastien, F-13400 Aubagne (FR); GIBELIN, Jérémy, F-83330 Le Beausset (FR)
(74) Mandataire: Derambure Conseil
(86) Numéro de dépôt international: PCT/FR2012/051662
(87) Numéro de publication internationale: WO 2013/011231

(56) Documents cités:
- FR-A1- 2 924 034
- US-A1- 2009 178 495

## Description

L'invention concerne un perfectionnement au raccordement d'un accessoire à un récipient.

Plus spécialement, elle a pour premier objet un dispositif de raccordement à un récipient d'un accessoire dont la partie proximale active est destinée à être mise en communication avec l'intérieur du récipient par une ouverture du récipient.

Elle a pour deuxième objet un ensemble pour la mesure ou le contrôle d'un paramètre d'un liquide se trouvant à l'intérieur d'un récipient au moyen d'un accessoire de mesure ou de contrôle, comprenant ledit dispositif de raccordement.

Elle a pour troisième objet un ensemble à récipient pour application biopharmaceutique avec mesure ou contrôle d'un paramètre d'un liquide se trouvant à l'intérieur du récipient, comprenant un tel dispositif de raccordement ou un tel ensemble pour la mesure ou le contrôle.

Elle a pour quatrième objet un procédé de pré assemblage d'un tel ensemble pour la mesure ou le contrôle.

Elle a pour cinquième objet un procédé de montage d'un tel ensemble à récipient.

Elle a pour sixième objet un procédé de mise en oeuvre d'un tel ensemble pour la mesure ou le contrôle.

Le récipient en question est en l'espèce un récipient de stockage et/ou de traitement d'un contenu tel qu'un produit biopharmaceutique. Un tel récipient s'entend en l'espèce d'un récipient rigide réutilisable ou d'un récipient souple à usage unique tel qu'une poche.

Cette poche peut être une poche dite 2D, telle que celle commercialisée par Sartorius Stedim Biotech sous la marque Flexboy®, dont le volume, typiquement, peut être compris entre 50 millilitres et 50 litres.

Cette poche peut également être un récipient souple tel que décrit dans le document WO 00/04131 dont le volume peut dépasser, et généralement dépasse, 50 litres. Un tel récipient est couramment appelé poche 3D et est commercialisé par Sartorius Stedim Biotech sous la marque Flexel®. Il est alors connu qu'à cette poche sont associés des moyens rigides de maintien extérieur de la poche, au moins lorsqu'elle comporte son contenu.

Un produit biopharmaceutique s'entend en l'espèce d'un (ou plusieurs) produit issu de la biotechnologie - milieux de cultures, cultures cellulaires, solutions tampon, liquides de nutrition artificielle - ou un produit pharmaceutique ou plus généralement un produit destiné à être utilisé dans le domaine médical. Un tel produit est sous forme liquide, pâteuse, poudreuse, en une ou plusieurs phases, homogènes ou non, apte à s'écouler à travers une valve, raison pour laquelle le produit peut, dans le contexte de l'invention, être qualifié de fluide. L'invention s'applique également à des produits autres que biopharmaceutiques, selon la définition qui vient d'en être donnée, mais qui sont soumis à des exigences analogues en ce qui concerne leur stockage ou leur traitement.

L'accessoire s'entend en l'espèce d'une sonde pour mesurer des paramètres relatifs au contenu du récipient, comme la pression, le pH, la température, la colorimétrie, la conductimétrie, etc., ou encore un tube de remplissage ou un tube de vidange. L'invention s'applique également à des accessoires autres, mais qui ont vocation à être raccordés à un récipient en étant mis en communication avec l'intérieur de celui-ci.

Dans le domaine de la biopharmacie, il est usuel d'utiliser les récipients comme lieu pour effectuer des réactions chimiques ou biologiques, et le cas échéant les suivre et/ou les contrôler, ou encore comme moyen de stockage. Afin d'éviter la pénétration de germes à l'intérieur du récipient, il importe que l'environnement soit clos, stérile et aseptique, sans contact avec le milieu extérieur.

Les réactions doivent, en général, se dérouler dans des conditions déterminées et contrôlées (pression, pH, température, colorimétrie, conductimétrie, etc.) ou le stockage réalisé dans des conditions maîtrisées. Il est donc nécessaire d'effectuer plus ou moins fréquemment des mesures ou des contrôles de paramètres caractérisant le produit contenu dans le récipient. Ces mesures doivent être effectuées dans des conditions aseptiques, pour les raisons indiquées précédemment.

Le document DE 10 2004 015703 décrit un dispositif de raccordement comprenant deux éléments pourvus de moyens de guidage et de crochets.

Le document DE 42 07 845 décrit un dispositif de support d'électrode d'analyse, comportant un tube extérieur fixe, un tube intérieur mobile à coulissement et un ensemble monté transversalement incluant un piston monté dans un carter, cet ensemble pouvant former une entrée étanche pour le tube intérieur, de manière à pouvoir retirer l'électrode du récipient dans lequel elle est destinée à être placée, sans perdre le contenu de celui-ci.

Le document WO 86/07 151 décrit un dispositif de maintenance pour le calibrage et le nettoyage automatique d'une sonde faisant partie d'un système de mesure en continu de processus chimique ou biologique, consistant à analyser des paramètres du fluide à investiguer.

Le document DE 25 57 542 décrit un dispositif de mesure pour des valeurs électriques de milieux s'écoulant, avec des détecteurs de mesure disposés dans des enceintes de passage, dans lequel le passage d'un corps de soupape forme l'enceinte de passage et le détecteur est monté dans ce dernier.

Le document US2005/0239198 décrit un raccordement stérile et aseptique au moyen de deux éléments, un premier élément fixé sur le récipient et un deuxième élément apte à être raccordé sur le premier élément. Le premier élément comporte, à son extrémité libre, un moyen de connexion pourvu d'un couvercle. Le second élément comprend une sonde disposée à l'intérieur d'un manchon flexible pourvu d'un moyen de connexion pourvu d'un couvercle, le moyen de connexion du deuxième élément étant destiné à être connecté au moyen de connexion du premier élément après retrait de leur couvercle respectif. La sonde est fixée au récipient en glissant le moyen de connexion du deuxième élément dans le moyen de connexion du premier élément. L'extrémité de la sonde est ensuite insérée à l'intérieur du récipient par compression du manchon, la sonde glissant à l'intérieur du premier élément jusqu'à atteindre l'intérieur du récipient.

Le document US 2009/0178495 décrit une vanne d'échantillonnage avec laquelle des échantillons contenant des matières sensibles mécaniquement sont enlevés d'une manière stérile d'un bioréacteur,

Le document WO 2009/071829 décrit un dispositif de raccordement à un récipient d'un accessoire dont la partie proximale active est apte à être mis en communication avec l'intérieur du récipient par l'intermédiaire d'une ouverture du récipient, le dispositif comprenant un premier élément, un deuxième élément, un troisième élément et une chambre, dans lequel :
▪ le premier élément a pour fonctions d'assurer la fixation rigide du dispositif de raccordement au récipient, de définir une voie de passage, entre l'intérieur et l'extérieur du récipient, de servir de support pour le deuxième élément et de guide en translation axiale sur une course axiale C dont les deux fins de course correspondent à un premier état distal et à un second état proximal, de contribuer à servir de guide pour le troisième élément, de contribuer à délimiter la chambre,
▪ le deuxième élément a pour fonctions de servir de support au troisième élément, lequel est fixé rigidement sur lui, de former un élément mobile à coulissement axial sur la course C dont les deux fins de course correspondent au premier état distal et au second état proximal,
▪ le troisième élément, auquel appartient une tête a pour fonctions de servir de support à l'accessoire, lequel est fixé rigidement sur lui, de former un élément mobile à coulissement axial sur la course C dont les deux fins de course, correspondent au premier état distal et au second état proximal, dans le premier état distal, d'assurer, grâce à la tête, la fermeture étanche de l'ouverture, dans le second état proximal, d'assurer, grâce à la tête, une protection de la partie proximale active, de contribuer à délimiter la chambre,
▪ la chambre est dans le premier état distal fermée de façon étanche et, dans le second état proximal, en communication avec l'intérieur et ayant pour fonction d'y loger à l'intérieur ou d'y placer en périphérie la partie proximale active, et, le cas échéant, d'y mettre un liquide de stockage, d'y mettre un liquide de conservation, réglage, contrôle, ou calibration de l'accessoire ou de constituer une chambre fermée de façon étanche dans lequel est placé l'accessoire en attente d'utilisation.

Selon la description détaillée d'un mode particulier de réalisation d'un tel dispositif de raccordement, celui-ci comprend, outre l'accessoire, trois éléments constitués de trois pièces distinctes assemblées entre elles. La pièce formant le premier élément est de forme tubulaire avec à une extrémité un rebord transversal en forme de collerette destinée à être fixé directement sur la paroi du récipient autour de son ouverture. La pièce formant le troisième élément comporte une tête en forme de paroi transversale dont le chant périphérique comporte un décrochement apte à coopérer avec le siège d'appui formé par le rebord transversal de la première pièce, la tête ayant un diamètre plus grand que celui de l'ouverture du récipient.

Avec un tel mode particulier de réalisation, le procédé de montage du dispositif de raccordement, de l'accessoire et du récipient consiste aux opérations suivantes :
▪ on dispose d'un récipient vide de contenu, de l'accessoire, et des éléments constitutifs du dispositif de raccordement,
▪ on fixe rigidement le premier élément sur le récipient à l'endroit de l'ouverture,
▪ on insère l'accessoire dans le troisième élément et on le fixe rigidement à celui-ci,
▪ on monte l'ensemble troisième élément + accessoire sur et dans le deuxième élément,
▪ on monte l'ensemble deuxième élément + troisième élément + accessoire sur et dans le premier élément.

Un tel procédé de montage, qui d'une manière générale est mis en oeuvre en salle blanche, possède de ce fait, en autres, les inconvénients suivants ;
- un tel dispositif de raccordement est monté manuellement par un opérateur sur la ligne même de fabrication dudit récipient pharmaceutique ce qui nuit directement à la productivité.
- l'accessoire peut nécessiter la manipulation de liquide ce qui en salle blanche représente un fort risque de souillure des produits fabriqués. Par exemple si l'accessoire est une sonde Ph il sera nécessaire d'emplir la chambre avec un liquide pour éviter que la sonde Ph ne sèche.
- la manipulation des différents éléments et éventuellement la manipulation de liquide rendent ce procédé de montage fastidieux pour l'opérateur.

L'invention vise à perfectionner le dispositif de raccordement à un récipient d'un accessoire dont la partie proximale active est apte à être mis en communication avec l'intérieur du récipient par l'intermédiaire d'une ouverture du récipient du type enseigné par le document WO 2009/071829. Tout en gardant les avantages d'un tel dispositif, il s'agit de le simplifier, de permettre son pré assemblage et si nécessaire l'emplissage de sa chambre hors salle blanche, de permettre l'assemblage et le montage par un simple coulissement axial dans la direction proximale, et de garantir une étanchéité au niveau de la chambre tel qu'il est possible de prévoir une nouvelle calibration de l'accessoire tel qu'une sonde, en limitant le risque de contamination.

A cet effet, selon un premier aspect, l'invention vise un dispositif de raccordement étanche à un récipient d'un accessoire dont la partie proximale active est destinée à être mise en communication avec l'intérieur du récipient par une ouverture du récipient, le dispositif présentant un axe et comprenant :
▪ un premier moyen, de forme générale tubulaire avec une ouverture d'extrémité proximale et une ouverture d'extrémité distale, agencé de sorte à assurer la fixation du dispositif au récipient, à constituer une voie de passage entre l'intérieur et l'extérieur du récipient, à former un support de guidage à coulissement axial du second moyen entre un état distal fermé et un état proximal ouvert, sur une course axiale C, à contribuer à former une chambre,
▪ un second moyen, de forme générale allongée, monté axialement dans le premier moyen, avec interposition d'un organe d'étanchéité latéral, de sorte à pouvoir être déplacé à coulissement axial sur la course C entre l'état distal fermé et l'état proximal ouvert, agencé de sorte à comporter une tête transversale à sa partie extrême proximale qui est apte, à l'état distal fermé, à fermer de façon étanche l'ouverture, à comporter un espace ayant une paroi proximale formée par la tête adjacente, une limite distale, et une ouverture de passage latérale, à comporter à sa partie extrême distale une partie d'actionnement à coulissement axial, et à former un support de l'accessoire de sorte que sa partie active soit située dans ledit espace, à contribuer à former une chambre,
▪ une chambre, incluant ledit espace et qui, à l'état distal fermé, est fermée latéralement par le premier moyen et, le cas échéant, est apte à contenir, de façon étanche, un liquide de conservation, de réglage, de contrôle ou de calibration de l'accessoire, et qui, à l'état proximal ouvert, est dégagée du premier moyen et, ainsi est apte à être en communication avec l'intérieur par l'ouverture de passage latérale.

Ce dispositif est tel que
▪ le premier moyen comporte une partie d'extrémité proximale d'assemblage fixe par coulissement, agencée de sorte à pouvoir être assemblée de façon fixe et étanche, avec interposition d'un organe d'étanchéité latéral, sur une partie complémentaire d'assemblage fixe du récipient, moyennant au moins un coulissement axial dans la direction proximale,
▪ la tête transversale est de forme et de dimension latérale périphérique correspondant à celles de l'intrados du premier moyen, de sorte à être apte à coulisser axialement dans le premier moyen dans la direction proximale, depuis son ouverture distale jusqu'à sa partie extrême proximale, la tête étant garnie d'un organe d'étanchéité latéral de tête, tel qu'un joint de tête,
▪ le second moyen est apte à être pré assemblé au premier moyen moyennant un coulissement axial dans la direction proximale, le premier moyen et le second moyen ainsi pré assemblés formant un tout structurel apte à être assemblé à ladite partie complémentaire d'assemblage par un coulissement axial dans la direction proximale.

Selon des réalisations, à l'état distal fermé, la tête est située à ou adjacente à l'ouverture proximale du premier moyen, et/ou la partie d'actionnement est située écartée de la partie extrême distale du premier moyen, dans la direction distale, d'une distance au moins égale à la course C. Et, à l'état proximal ouvert, la tête est située écartée de l'ouverture proximale du premier moyen, dans la direction proximale, d'une distance au moins égale à la course C, et/ou la partie d'actionnement est située à, ou adjacente de, ou proche de, la partie extrême distale du premier moyen, en particulier hors du premier moyen.

Selon des réalisations, le premier moyen comprend, en particulier est constitué par, une première pièce unique et/ou le second moyen comprend, en particulier est constitué par, une seconde pièce unique, avec organes d'étanchéité latéraux rapportés.

Selon une réalisation, le premier moyen comporte une première pièce de forme générale tubulaire s'étendant en direction axiale sur une longueur plus grande que la course C, en particulier substantiellement plus grande que la course C, dont l'extrados est de forme et de dimension latérale périphérique correspondant à celles de l'intrados de ladite partie complémentaire d'assemblage, de sorte à être apte à être montée axialement dans ladite partie complémentaire d'assemblage, ladite première pièce ayant un rebord radial latéral vers l'extérieur apte à coopérer en blocage axial dans la direction proximale avec un bord de ladite partie complémentaire d'assemblage et/ou un organe d'étanchéité étant interposé entre l'extrados de ladite première pièce et l'intrados de ladite partie complémentaire d'assemblage, en particulier un joint porté par l'extrados de ladite première pièce.

Selon une réalisation, le second moyen comporte une seconde pièce de forme générale tubulaire s'étendant en direction axiale sur une longueur plus grande que celle du premier moyen, en particulier au moins égale à celle du premier moyen augmentée de la course C, et plus spécialement sur une longueur proche de celle du premier moyen augmentée de la course C. En particulier, le second moyen comporte une seconde pièce pourvue à sa partie extrême distale d'un rebord radial latéral vers l'extérieur apte à coopérer en blocage axial dans la direction proximale avec le bord de la partie extrême distale du premier moyen, formant le cas échéant partie d'actionnement.

Selon une réalisation, le second moyen est monté axialement dans le premier moyen avec interposition d'un ou plusieurs organes d'étanchéité latéraux sous la forme d'un ou plusieurs joints portés par l'extrados du second moyen. En particulier, un organe d'étanchéité distal et un organe d'étanchéité proximal écartés l'un de l'autre en direction axiale. En particulier, l'écartement en direction axiale entre l'organe d'étanchéité distal et l'organe d'étanchéité proximal est plus grand, en particulier légèrement plus grand, que l'écartement en direction axiale entre l'organe d'étanchéité proximal et l'organe d'étanchéité de tête. En particulier, l'écartement en direction axiale entre l'organe d'étanchéité proximal et l'organe d'étanchéité de tête est égal ou voisin de la course C.

Selon une réalisation, est ménagée dans le second moyen une conduite, tel qu'un perçage, de liquide, s'étendant axialement, associée fonctionnellement à l'accessoire en particulier en vue de sa conservation, son réglage, son contrôle ou sa calibration, ayant une ouverture d'extrémité proximale débouchant dans ledit espace, respectivement dans la dite chambre, et une ouverture d'extrémité distale débouchant vers la partie extrême distale du second moyen, agencée de sorte que la conduite soit apte à être prolongée en direction distale hors second moyen, par un organe de raccordement extérieur, tel que comprenant au moins un tube. En particulier, le dispositif de raccordement comporte au moins deux conduites respectivement d'amenée et de vidange.

Selon une réalisation, le premier moyen et le second moyen sont pré assemblés de sorte à former un tout structurel à l'état distal fermé. En particulier, d'origine, l'accessoire est fixé au second moyen, en particulier par surmoulage de la partie distale de l'accessoire, et/ou la chambre, fermée, est emplie d'un liquide de calibration de l'accessoire.

Selon un deuxième aspect, l'invention a pour objet un ensemble pour la mesure ou le contrôle d'un paramètre d'un liquide se trouvant à l'intérieur d'un récipient au moyen d'un accessoire de mesure ou de contrôle dont la partie proximale active est destinée à être mise en communication avec l'intérieur du récipient par une ouverture du récipient, ledit ensemble pour la mesure ou le contrôle comprenant ledit accessoire, un tel dispositif de raccordement tel qu'il a été décrit, et, le cas échéant, un liquide de calibration de l'accessoire emplissant la chambre fermée dudit dispositif de raccordement à l'état distal fermé, ledit ensemble pour la mesure ou le contrôle étant prêt à être monté sur un récipient.

Selon une réalisation dans laquelle le dispositif de raccordement est du type avec une ou plusieurs conduites, ledit ensemble pour la mesure ou le contrôle comporte également, associé à la conduite ou à chacune des conduites, par une liaison fixe et communicante, l'organe de raccordement extérieur, tel que comprenant au moins un tube et, le cas échéant un ou plusieurs organes de connexion, d'arrêt, de circulation, de filtration, de mesure, de réception pour l'amenée ou la collecte du liquide destiné à passer dans ledit organe de raccordement extérieur et ladite conduite associée.

Selon une autre réalisation, dans laquelle le dispositif de raccordement est du type dans lequel est ménagé dans le second moyen une ou plusieurs conduites, ledit ensemble pour la mesure ou le contrôle comporte également, associé à la conduite ou à chacune des conduites, par une liaison fixe et communicante, l'organe de raccordement extérieur, tel que comprenant au moins un tube et, un ou plusieurs organes d'échantillonnage apte à récupérer un échantillon du contenu du récipient emprisonné dans la chambre après le passage du dispositif de raccordement de l'état ouvert à l'état fermé.

Selon une réalisation, l'accessoire est une sonde de mesure du pH du contenu se trouvant à l'intérieur du récipient.

Selon un troisième aspect, l'invention a pour objet un ensemble à récipient pour application biopharmaceutique avec mesure ou contrôle d'un paramètre d'un liquide se trouvant à l'intérieur du récipient, comprenant un récipient ayant une paroi pourvue d'une ouverture et, fixée de façon rigide et étanche à ladite paroi autour de l'ouverture, une partie complémentaire d'assemblage fixe et étanche apte à coopérer avec la partie d'extrémité proximale d'assemblage fixe du premier moyen d'un dispositif de raccordement ou d'un ensemble pour la mesure ou le contrôle tel qu'il a été précédemment décrit.

Selon une réalisation, la partie complémentaire d'assemblage fixe et étanche a une forme générale tubulaire avec une extrémité proximale avec ouverture et une extrémité distale avec ouverture, et une collerette d'extrémité proximale apte à être solidarisée de manière fixe et étanche à la paroi du récipient autour de son ouverture.

Selon les réalisations, le récipient 2 est soit rigide et réutilisable soit souple et à usage unique, tel qu'une poche dite 2D ou 3D et, dans ce dernier cas, selon une réalisation, il est prévu des moyens rigides de maintien extérieur du récipient pourvus d'un passage pour le dispositif de raccordement ou l'ensemble pour la mesure ou le contrôle.

Selon un quatrième aspect, l'invention a pour objet un procédé de pré assemblage d'un ensemble pour la mesure ou le contrôle d'un paramètre tel qu'il a été décrit précédemment, en vue de son montage ultérieur sur un récipient, qui comprend la succession des étapes suivantes :
▪ on dispose de moyens aptes à constituer un dispositif de raccordement tel que précédemment décrit,
▪ on dispose d'un accessoire de mesure ou de contrôle,
▪ on agence le second moyen du dispositif de raccordement et l'accessoire, pour que le second moyen supporte l'accessoire de façon que sa partie active soit située dans ledit espace ménagé par le second moyen,
▪ on fait coulisser axialement le second moyen dans le premier moyen depuis son ouverture d'extrémité distale en direction proximale, jusqu'à l'état distal fermé.

Selon des réalisations, à l'état distal fermé, la chambre fermée est emplie d'un liquide de conservation et/ou calibration de l'accessoire, et le procédé est réalisé sans la nécessité d'être confiné dans une salle blanche.

Selon un cinquième aspect, l'invention a pour objet un procédé de montage d'un ensemble à récipient tel que précédemment décrit, qui comprend la succession des étapes suivantes :
▪ on dispose d'un récipient vide de contenu, pourvu, autour d'une ouverture d'une partie complémentaire d'assemblage avec un dispositif de raccordement,
▪ on dispose d'un ensemble pour la mesure ou le contrôle d'un paramètre tel que précédemment décrit,
▪ on fait coulisser axialement l'ensemble pour la mesure ou le contrôle d'un paramètre dans ladite partie complémentaire d'assemblage depuis son ouverture d'extrémité distale en direction proximale, jusqu'à l'état distal fermé.

Selon une réalisation, le procédé de montage d'un ensemble à récipient comprend une étape ultérieure de stérilisation de l'ensemble à récipient.

Selon un sixième aspect, l'invention a pour objet un procédé de mise en oeuvre d'un ensemble pour la mesure ou le contrôle d'un paramètre faisant partie d'un ensemble à récipient monté tel que précédemment décrit, qui comprend la succession des étapes suivantes :
▪ la chambre étant vide, on fait coulisser axialement le second moyen du dispositif de raccordement de l'état distal fermé à l'état proximal ouvert, dans la direction proximale,
▪ on met en oeuvre l'accessoire.

Selon une réalisation, le procédé de mise en oeuvre comporte au moins une opération supplémentaire de conservation, de réglage, de contrôle ou de calibration de l'accessoire, en particulier une opération initiale avant que le récipient soit empli de son contenu, une opération finale, après que le récipient a été vidangé de son contenu, et le cas échéant, une opération après que le récipient a été empli de son contenu et avant qu'il a été vidangé de son contenu, alors que le l'ensemble pour la mesure ou le contrôle d'un paramètre et son dispositif de raccordement sont à l'état distal fermé et que la chambre est fermée.

L'invention permet en particulier de pouvoir stocker, calibrer et utiliser une sonde à usage unique, dans des conditions stériles ou non.

L'invention est tout particulièrement applicable dans le domaine biopharmaceutique, par exemple pour des applications liées au mélange, pour les bioréacteurs à usage unique, pour la thermorégulation.

On décrit maintenant un mode particulier de réalisation de l'invention à l'aide des dessins, dans lesquels :
- la figure 1 est une vue en coupe axiale illustrant en situation de montage sur un réservoir représenté de façon partielle, le dispositif de raccordement et l'accessoire représenté de façon schématique et étant ici une sonde, le dispositif de raccordement et l'ensemble pour la mesure ou le contrôle d'un paramètre étant dans l'état distal fermé correspondant à une situation de stockage et/ou de calibration,
- la figure 2 est une vue analogue à celle de la figure 1, à l'état proximal ouvert correspondant à une situation d'utilisation de la sonde.

Un dispositif 1 est destiné au raccordement à un récipient 2 à usage biopharmaceutique d'un accessoire 3 apte à être mis en communication avec l'intérieur 4 du récipient 2 et son contenu, tel qu'un produit biopharmaceutique fluide comme défini. Un tel dispositif 1 perfectionne celui décrit dans le document WO 2009/071829 dont il est tenu compte de l'enseignement.

On entend par « raccorder », le fait qu'originellement, le récipient 2 et l'accessoire 3 sont totalement distincts et séparés et que, grâce au dispositif 1, l'accessoire 3 est joint et relié au récipient 2 et qu'un lien fonctionnel peut être établi entre le récipient 2, et son contenu, et l'accessoire 3.

On dénomme « ensemble pour la mesure ou le contrôle », l'ensemble assemblé 1 + 3, comprenant le dispositif 1 de raccordement et l'accessoire 3 et, le cas échéant, un liquide de calibration de l'accessoire 3 emplissant la chambre 18 fermée dudit dispositif 1 de raccordement à l'état distal fermé. Un tel ensemble 1 + 3 pour la mesure ou le contrôle est prêt à être monté sur un récipient 2.

On dénomme « ensemble à récipient » ou encore « conteneur fonctionnel », un ensemble 1 + 2 + 3 monté, comprenant un récipient 2 pour une application biopharmaceutique, avec mesure ou contrôle d'un paramètre d'un liquide se trouvant à l'intérieur 4 du récipient 2, le récipient 2 ayant une paroi 6 pourvue d'une ouverture 7 et, fixée de façon rigide et étanche à ladite paroi 6 autour de l'ouverture 7, une partie complémentaire d'assemblage fixe et étanche 19 apte à coopérer avec une partie d'extrémité proximale 32 d'assemblage fixe d'un premier moyen 15 (en l'espèce une première pièce 15) du dispositif 1 de raccordement ou d'un ensemble 1 + 3 pour la mesure ou le contrôle. Un tel ensemble 1 + 2 + 3 à récipient constitue conteneur fonctionnel est destiné au stockage et/ou au traitement d'un produit biopharmaceutique, désigné par la suite le contenu. Selon les réalisations envisagées, le récipient 2 est rigide et réutilisable ou il est souple et à usage unique tel qu'une poche telle qu'une poche dite 2D ou une poche dite 3D, comme il a été défini plus haut.

Dans une réalisation, une telle poche 3D a une forme générale parallélépipédique, notamment cubique ou sensiblement cubique, ayant une paroi périphérique 6, formant au moins le fond et la partie latérale, et le plus souvent la partie supérieure, de manière que le récipient 2 soit de type fermé, et un ou plusieurs ports appropriés à la destination, ménagés sur la paroi 6. Dans une réalisation correspondant à un grand volume qui dépasse 50 I et peut atteindre 3.000 I, le récipient 2 est pliable.

Avec un tel récipient 2 souple, il est prévu que le conteneur fonctionnel inclut en outre des moyens rigides de maintien extérieur. Ces moyens, dont l'existence est connue en soi, ne sont pas représentés. Il peut s'agir notamment d'un bac métallique ayant des parois pleines ou non.

La paroi 6 du récipient 2 est pourvue d'une l'ouverture 7 destinée au montage, transversalement par rapport à la paroi 6, du dispositif 1 de raccordement et de l'accessoire 3, de manière que ce dernier puisse être mis en communication avec l'intérieur 4 du récipient 2 et notamment à être mis en contact avec le contenu du récipient 2, alors même que l'accessoire 3 est situé au moins substantiellement dans l'extérieur 8 du récipient 2.

Ce qui vient d'être décrit dans le cas d'une poche 3D peut être transposé au cas d'une poche 2D et au cas d'un récipient 2 rigide.

Le dispositif 1, comme le plus souvent l'accessoire 3, présente un axe longitudinal 1a disposé au moins sensiblement perpendiculairement à la paroi 6.

L'intérieur 4 du récipient 2 est défini conventionnellement comme la zone interne au récipient 2, délimitée par l'enveloppe de la paroi 6, située du côté où se trouve la plus grande partie du contenu. L'extérieur 8 du récipient 2 est défini comme la zone se trouvant de l'autre côté de l'enveloppe de la paroi 6.

Les moyens rigides de maintien extérieur du récipient 2, lorsqu'ils sont prévus, comportent un passage pour le dispositif 1 de raccordement, situé en regard ou sensiblement en regard de l'ouverture 7.

Le terme « accessoire » signifie non pas que l'objet 3 qu'il désigne serait secondaire, ou simplement optionnel, mais que cet objet 3 est complémentaire et dans la dépendance étroite du récipient 2 et du contenu de celui-ci, en ayant une fonction accompagnant celle du récipient 2.

Dans le cadre de l'invention, l'accessoire 3 est préférentiellement à usage unique, tout comme le dispositif 1 de raccordement.

Dans la réalisation représentée, l'accessoire 3 est une sonde comportant une partie proximale active 9 apte à mesurer, détecter, contrôler, enregistrer (par la suite mesurer) un ou plusieurs paramètres utiles se rapportant au contenu du récipient 2, comme notamment la pression, le pH, la température, la colorimétrie, la conductimétrie.

De façon générale, l'accessoire 3 comporte une enveloppe extérieure de forme plus ou moins complexe, allongée, notamment de révolution à base circulaire, avec la partie proximale active 9 et une partie extrême distale 10. A cette partie extrême distale 10 peuvent être associés des moyens tels que conducteur électrique, tube...

Dans la réalisation représentée, l'accessoire 3 est disposé centralement par rapport à l'axe 1a.

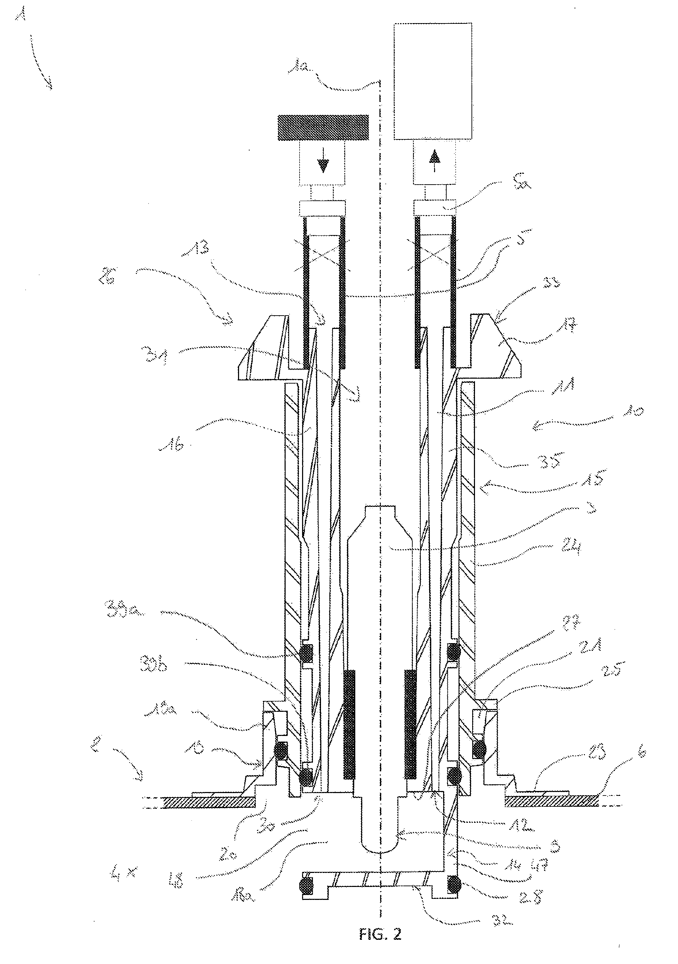

La partie active 9 de l'accessoire 3 est apte, lorsque nécessaire ou souhaité, à être mise en communication avec l'intérieur 4 du récipient 2, et plus spécialement à être mise en contact avec le contenu du récipient 2, afin de pouvoir jouer sa fonction de mesure. Dans ce cas, il est prévu, comme représenté sur la figure 2, que la partie proximale active 9 est apte à être introduite dans l'intérieur 4 du récipient 2, en franchissant l'ouverture 7 de la paroi 6, pour aller au-delà de cette dernière, de manière à être en contact intime avec le contenu, dans une zone où celui-ci est homogène ou du moins suffisamment homogène, en ce qui concerne le paramètre en cause, avec le reste du contenu du récipient 2. L'ouverture 7 est alors préférentiellement située sur ou vers le fond du récipient 2, c'est-à-dire de manière à se trouver à un niveau plus bas que le niveau du contenu du récipient 2, même lorsque la quantité de contenu est faible et le niveau proche du fond du récipient 2.

Dans une autre réalisation, non représentée, la partie proximale active 9 de l'accessoire 3 est apte non pas, à proprement parler, à être introduite dans l'intérieur 4 du récipient 2, mais à être amenée en-deçà de la paroi 6 et à proximité de l'ouverture 7 sans toutefois la franchir, mais tout en étant en communication avec l'intérieur 4 du récipient 2. Dans une autre réalisation, non représentée, la partie proximale active 9 est apte à être amenée dans l'ouverture 7, dans le plan de la paroi 6, de manière à être en communication avec l'intérieur 4 du récipient 2.

Dans ces différentes réalisations, situations et positions, la partie proximale active 9 de l'accessoire 3 (et-l'accessoire 3 lui-même) est qualifiée d'insérée dans le récipient 2 ou dans l'intérieur 4 du récipient 2.

Dans d'autres réalisations, pouvant être combinées avec les précédentes, il est prévu non pas un seul, mais plusieurs, accessoires 3, tels que des sondes, soit pour plusieurs paramètres soit à différents endroits, notamment hauteur, du récipient 2.

Selon une seconde réalisation non représentée, l'accessoire 3 est un tube d'amenée et/ou un tube de vidange, du contenu du récipient 2 ou d'un ou plusieurs composants de celui-ci. Ce tube comporte une partie proximale active 9 vers le récipient 2 consistant en une ouverture d'amenée si le tube est un, ou fait fonction de, tube d'amenée, en une ouverture de vidange si le tube est un, ou fait fonction de, tube de vidange, cette ouverture étant classiquement située à l'extrémité libre du tube. La partie proximale active 9 du tube est apte, lorsque nécessaire, à être mise en communication avec l'intérieur 4 du récipient 2, afin de pouvoir jouer sa fonction d'amenée et/ou de vidange. La partie proximale active 9 du tube est alors apte soit à être amenée soit dans l'ouverture 7, dans le plan de la paroi 6 soit à être introduite dans l'intérieur 4 du récipient 2, en franchissant l'ouverture 7 de la paroi 6 soit à être amenée en-deçà de la paroi 6 et à proximité de l'ouverture 7 sans toutefois la franchir. Ces différentes réalisations, situations et positions, correspondent à ce qui a été défini comme l'insertion de la partie proximale active 9 dans le récipient 2 ou dans l'intérieur 4 du récipient 2. Lorsque l'accessoire 3 est un tube d'amenée et/ou un tube de vidange, l'ouverture 7 est située par rapport au fond du récipient 2 à la hauteur appropriée à la destination du tube, soit à un niveau proche du fond du récipient 2 soit au contraire à un niveau plus ou moins éloigné du fond.

Selon la réalisation représentée dans le cas où l'accessoire 3 est une sonde pH, il est prévu que le dispositif 1 de raccordement et plus spécialement un second moyen 16 (en l'espèce une seconde pièce 16) ménage une conduite 11, et plus particulièrement au moins deux conduites 11 respectivement d'amenée et de vidange. Une telle conduite 11 de liquide, tel qu'un perçage, s'étend axialement et est associée fonctionnellement à l'accessoire 3 en particulier en vue de , sa conservation, son réglage, son contrôle ou sa calibration. Une telle conduite 11 est placée à côté ou à proximité de l'accessoire 3, en étant excentrée par rapport à lui. Une telle conduite 11 a une ouverture d'extrémité proximale 12 débouchant dans un espace 18a du dispositif 1 de raccordement, respectivement dans la chambre 18, et une ouverture d'extrémité distale 13 débouchant vers la partie extrême distale 26 du second moyen (seconde pièce) 16. La conduite 11 est apte à être prolongée en direction distale, hors du second moyen (seconde pièce) 16, par un organe de raccordement extérieur 5, tel que comprenant au moins un tube. En outre, il peut être prévu, associé à la conduite 11 ou à chacune des conduites 11, par une liaison fixe et communicante, l'organe de raccordement extérieur 5, tel que comprenant au moins un tube et, le cas échéant un ou plusieurs organes 5a de connexion - par exemple de type Luer, d'arrêt - par exemple un clamp, de circulation, de filtration, de mesure, de réception pour l'amenée ou la collecte du liquide destiné à passer dans ledit organe de raccordement extérieur 5 et ladite conduite associée 11.

Plus généralement, Il peut aussi être envisagé d'utiliser un organe 5a d'échantillonnage permettant de récupérer par aspiration au travers de l'organe de raccordement extérieur 5 - ou tout autre processus analogue - un échantillon du contenu biopharmaceutique emprisonné dans la chambre après que le dispositif de raccordement selon l'invention soit passé de l'état ouvert à l'état fermé.

Le terme « proximal » se réfère conventionnellement à ce qui, de façon relative, est proche, ou plus proche, du récipient 2, notamment de son intérieur 4. Le terme « distal » se réfère inversement à ce qui, de façon relative, est éloigné, ou plus éloigné, du récipient 2, notamment de son intérieur 4. Ces qualificatifs sont utilisés pour repérer les parties constitutives du dispositif 1 de raccordement, de l'accessoire 3...Il est entendu toutefois que le dispositif 1 de raccordement ou l'accessoire 3 ou la partie fonctionnelle 11 peut être considéré en soi, indépendamment du récipient 2, avant montage sur celui-ci. Ces qualificatifs sont utilisés également pour repérer la localisation des parties constitutives du dispositif 1 de l'accessoire 3 .. par rapport au récipient 2 ou pour repérer leur position par rapport à celui-ci, si cette position n'est pas fixe. Ces qualificatifs sont utilisés enfin, conventionnellement, pour distinguer les deux états dans lesquels peuvent se trouver le dispositif 1 de raccordement, l'accessoire 3, l'ensemble 1 + 3 pour la mesure et le contrôle et l'ensemble 1 + 2 + 3 à récipient.

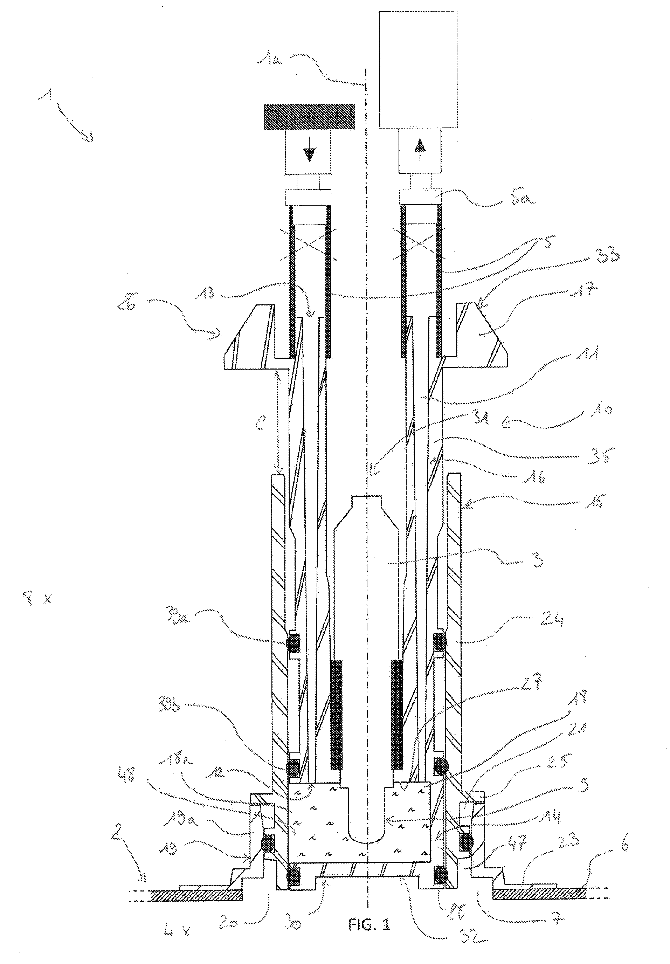

En effet, le dispositif 1 de raccordement, l'accessoire 3 et notamment sa partie proximale active 9, l'ensemble 1 + 3 pour la mesure et le contrôle et l'ensemble 1 + 2 + 3 à récipient peuvent se trouver dans l'un de deux états extrêmes possibles correspondant à deux positions relatives par rapport au récipient 2, à deux manières d'être, fonctions, ou possibilité d'action. Ces états se comprennent lorsque le dispositif 1 de raccordement et l'accessoire 3 sont montés sur le récipient 2. Mais, comme précédemment, il est entendu toutefois que le dispositif 1 de raccordement ou l'accessoire 3 peut être considéré en soi, indépendamment du récipient 2, avant montage sur celui-ci, les deux états pouvant donc s'appliquer à eux, pris en tant que tels. Ces deux états sont illustrés, respectivement, par la figure 1 et la figure 2.

Les deux états extrêmes possibles sont un premier état qualifié de distal (figure 1) et un second état qualifié de proximal (figure 2). Les qualificatifs de « premier » et « second » expriment l'idée qu'au départ, on se trouve dans l'état distal fermé. L'état distal est un état fermé et l'état proximal est un état ouvert.

Dans l'état distal fermé (figure 1), la partie proximale active 9 de l'accessoire 3 est totalement séparée, et de façon étanche, de l'intérieur 4 du récipient 2 par une tête 14 formant barrière, faisant partie du dispositif 1 de raccordement et plus précisément de son second moyen (seconde pièce) 16. Plus précisément, à l'état distal fermé, la tête 14 est située à ou adjacente à l'ouverture d'extrémité proximale 30 du premier moyen 15, et une partie d'actionnement 17 à la partie extrême distale 26 du second moyen (seconde pièce) 16, formée par un rebord radial latéral vers l'extérieur, est située écartée de l'extrémité de la partie extrême distale 33 du premier moyen (première pièce) 15, dans la direction distale, d'une distance au moins égale à la course C.

Dans l'état proximal ouvert (figure 2), la partie proximale active 9 de l'accessoire 3 est en communication avec l'intérieur 4 et insérée dans le récipient 2, de sorte à être en contact avec le contenu du récipient 2, ce qui permet à l'accessoire 3 d'assurer sa fonction, notamment dans le cas d'une sonde 3, de mesurer ou contrôler un paramètre du contenu du récipient 2. La tête 14 forme alors une protection de la partie proximale active 9 contre par exemple des produits solides se déplaçant à l'intérieur 4 du récipient 2 ou des organes en mouvements tels que faisant partis d'un mélangeur. Plus précisément, à l'état proximal ouvert, la tête 14 est située écartée de la partie extrême proximale 32 du premier moyen (première pièce) 15, dans la direction proximale, d'une distance au moins égale à la course C, et la partie d'actionnement 17 est située à, ou adjacente de, ou proche de, la partie extrême distale 31 du premier moyen (première pièce) 15, en particulier hors du premier moyen (première pièce) 15, notamment de sorte à être apte à coopérer en blocage axial dans la direction proximale avec le bord de la partie extrême distale 33 du premier moyen 15.

Dans la réalisation représentée correspondant au cas où l'accessoire 3 est une sonde 3, l'état distal fermé (figure 1) est typiquement celui correspondant au stockage ou à la calibration, tandis que l'état proximal ouvert (figure 2) est typiquement celui de la mesure, pour le contenu du récipient 2, du paramètre au moyen de l'accessoire 3 mis en oeuvre à cet effet.

Dans le cas non représenté où l'accessoire 3 est un tube, l'état distal fermé est typiquement celui correspondant au stockage ou à l'inutilisation de la fonction remplissage ou vidange du tube, tandis que l'état proximal ouvert est celui de la fonction remplissage ou vidange du tube.

Le dispositif 1 de raccordement comprend un premier moyen 15 (une première pièce 15) et un second moyen 16 (une seconde pièce 16). Le premier moyen 15 et le second moyen 16 sont assemblés l'un avec l'autre. L'accessoire 3 et le cas échéant la conduite 11 sont prévus sur le second moyen 16. Le dispositif 1 de raccordement comprend en outre un espace 18a et une chambre 18.

Le premier moyen (première pièce) 15 a plusieurs fonctions :
- Il assure la fixation rigide du dispositif 1 de raccordement au récipient 2, par l'intermédiaire de la partie d'assemblage fixe 19, qui fait partie du récipient 2.
- Il définit une voie de passage, entre l'intérieur 4 et l'extérieur 8 du récipient 2.
- Pour le second moyen (seconde pièce) 16, il sert de support et de guide à coulissement axial le long de l'axe 1a, sur une course C donnée dont les deux fins de course, correspondent respectivement au premier état distal fermé et au second état proximal ouvert.
- Il contribue à délimiter la chambre 18.

Le second moyen (seconde pièce) 16 a également plusieurs fonctions :
- Il forme un élément mobile à coulissement axial le long de l'axe 1a sur la course C dont les deux fins de course, correspondent respectivement au premier état distal fermé et au second état proximal ouvert.
- Il sert de support à l'accessoire 3, et le cas échéant à la conduite 11, tel qu'un perçage.
- Dans l'état distal fermé, il assure, grâce à la tête 14 la fermeture étanche de l'ouverture 7 en venant fermer avec étanchéité l'ouverture d'extrémité proximale 30 du premier élément 15, en formant ainsi une barrière interdisant l'accès à l'intérieur 4 du récipient 2 et la communication entre l'accessoire 3 et l'intérieur 4 du récipient 2.
- Dans l'état proximal ouvert, il assure, grâce à la tête 14 une protection de la partie proximale active 9 de l'accessoire 3, surtout lorsqu'elle est introduite à proprement parler dans l'intérieur 4 du récipient 2.
- Il contribue également à délimiter la chambre 18.

Dans la réalisation représentée, le premier moyen 15 comprend, et plus précisément est constitué par, une première pièce 15 unique et le second moyen 16 comprend, et plus précisément est constitué par, une seconde pièce unique 16, avec organes d'étanchéité latéraux interposés et rapportés 39a, 39b. Bien entendu, l'invention s'étend à des réalisations différentes en plusieurs pièces solidarisées entre elles, cette réalisation étant considérée comme équivalente. Les pièces 15 et 16 sont réalisées par exemple en ou à base de matière plastique ou comportent de la matière plastique ou certaines parties sont réalisées en silicone.

Le premier moyen 15 (première pièce 15), le second moyen 16 (seconde pièce 16), la partie complémentaire d'assemblage fixe et étanche 19 sont de forme tubulaire apte à permettre leur montage comme indiqué. Si ces trois pièces 15, 16 et 19 sont destinées à être déplacées l'une par rapport à l'autre par coulissement axial, elles sont de forme prismatique ou cylindrique. Si elles sont destinées à être déplacées l'une par rapport à l'autre par coulissement axial et par rotation autour de l'axe, elles sont de forme cylindrique circulaire.

On décrit maintenant plus spécialement la partie complémentaire d'assemblage fixe et étanche 19. La partie complémentaire d'assemblage 19 peut être considérée comme faisant partie du récipient 2, pour y être fixée de façon rigide et étanche autour de son ouverture 7. La partie complémentaire d'assemblage 19 ne fait pas en soi partie du dispositif 1 de raccordement, bien qu'elle soit indispensable au dispositif 1 de raccordement.

La partie complémentaire d'assemblage 19, rigide et ici monobloc, a une forme générale tubulaire d'axe 1a, avec une paroi latérale cylindrique ou prismatique 19a, ayant une extrémité proximale avec une ouverture d'extrémité proximale 20 et une extrémité distale avec une ouverture d'extrémité distale 21, et une collerette 23 d'extrémité proximale, annulaire, transversale, dirigée latéralement vers l'extérieur avec une étendue radiale suffisante, apte à être solidarisée de manière fixe et étanche à la paroi 6 du récipient 2 autour de son ouverture 7 par exemple par soudage par ultrasons, collage ou d'une autre manière analogue. Le diamètre de la paroi latérale tubulaire 19a est en correspondance avec le diamètre de l'ouverture 7 et en correspondance avec le diamètre du premier moyen (première pièce) 15, de manière que le premier moyen (première pièce) 15 puisse venir s'ajuster dans la partie complémentaire d'assemblage 19. La longueur axiale de la partie complémentaire d'assemblage 19 et donc de la paroi latérale tubulaire 19a est suffisante pour assurer le maintien du premier moyen (première pièce) 15 et donc du dispositif 1 de raccordement. Par exemple, la longueur axiale de la partie complémentaire d'assemblage 19 et de la paroi latérale tubulaire 19a peut être de l'ordre de la course C. Grace à sa forme tubulaire avec ses deux extrémités ouvertes proximale 20 et distale 21, la partie complémentaire d'assemblage 19 forme une voie de passage pour le contenu du récipient 2 depuis l'intérieur 4 et elle permet le passage, tant vers l'extrémité ouverte distale 21 que l'extrémité ouverte proximale 20, du dispositif 1 de raccordement.

On décrit maintenant plus spécialement la première pièce 15 formant le premier moyen 15.

La première pièce 15, rigide, est de forme générale tubulaire d'axe 1a, comportant une paroi tubulaire 24, une partie extrême proximale 32 ayant une ouverture d'extrémité proximale 30 et une partie extrême distale 33 ayant une ouverture d'extrémité distale 31, et s'étend en direction axiale sur une longueur plus grande que la course C, en particulier substantiellement plus grande que la course C, cela pouvant aller de trois à six fois la course C, par exemple, ces valeurs n'étant cependant pas limitatives.

L'extrados de la paroi tubulaire 24 est de forme et de dimension latérale périphérique correspondant à celles de l'intrados de ladite partie complémentaire d'assemblage 19. Ainsi, la première pièce 15 peut être montée axialement dans la partie complémentaire d'assemblage 19. L'intrados de la paroi tubulaire 24 correspond au jeu nécessaire près, moyennant la présence d'organes d'étanchéité 39a, 39b, interposés, au diamètre extérieur de la seconde pièce 16.

La première pièce 15 a un rebord radial latéral 25, vers l'extérieur, apte à coopérer en blocage axial dans la direction proximale avec le bord de l'extrémité distale 21 de la partie complémentaire d'assemblage 19, de sorte que, comme représenté, la partie extrême proximale 32 de la première pièce 15 vienne au droit, ou au voisinage, de la collerette 23 de la partie complémentaire d'assemblage 19.

Un organe d'étanchéité latéral 22 est interposé entre l'extrados de la première pièce 15 et l'intrados de la partie complémentaire d'assemblage 19. Cet organe d'étanchéité 22 est en particulier un joint torique en silicone ou autre matériau équivalent porté par l'extrados de ladite première pièce 15, dans une gorge ménagée à cet effet.

Ainsi, la première pièce 15 constitue une voie de passage entre l'intérieur 4 et l'extérieur 8 du récipient 2. D'autre part, la première pièce 15 comporte vers sa partie extrême proximale 32, une partie d'extrémité d'assemblage fixe par coulissement, pouvant être assemblée de façon fixe et étanche, avec interposition de l'organe d'étanchéité latéral 22, sur la partie complémentaire d'assemblage fixe 19 du récipient 2. Cet assemblage est réalisé par au moins un coulissement axial dans la direction proximale. Egalement, la première pièce 15 forme par l'intrados de sa paroi tubulaire 24 un support de guidage à coulissement axial de la seconde pièce 16 entre l'état distal fermé et l'état proximal ouvert, l'ouverture d'extrémité distale 31 permettant le montage de la seconde pièce 16 dans la première pièce 15, et l'ouverture d'extrémité proximale 30 permettant le passage et la sortie de la seconde pièce 16 et de l'accessoire 3 vers l'intérieur 4. Et, enfin, la première pièce 15 contribuer à former une chambre 18. Dans la réalisation représentée, la partie extrême distale 33 forme également butée pour la partie d'actionnement 17 de la seconde pièce 16 à l'état proximal ouvert.

La première pièce 15 et la partie complémentaire d'assemblage 19 sont mutuellement agencées de sorte que la partie complémentaire d'assemblage 19 assure un maintien approprié de la première pièce 15. Un tel maintien peut résulter d'un enfoncement à force. Il peut aussi résulter de la coopération d'organes complémentaires prévus sur la première pièce 15 et la partie complémentaire d'assemblage 19, tels qu'un moyen de liaison cinématique de type hélicoïdal, tel qu'un pivotement axial relatif imprimé à la première pièce 15 entraîne son coulissement axial de coopération avec la partie complémentaire d'assemblage 19. Un tel moyen de liaison cinématique de type hélicoïdal peut, en particulier, comporter un ergot latéral d'une pièce coopérant avec une gorge hélicoïdale de l'autre pièce.

On décrit maintenant plus spécialement la seconde pièce 16 formant le second moyen 16.

La seconde pièce 16, rigide, est de forme générale allongée, montée axialement par rapport à l'axe 1a, dans la première pièce 15, avec interposition d'organes d'étanchéité latéraux 39a, 39b. La seconde pièce 16 est ainsi apte à pouvoir être déplacée, par rapport à la première pièce 15 et à ce qui en est fixement solidaire tel que la partie complémentaire d'assemblage 19 et le récipient 2, à coulissement axial sur la course C entre l'état distal fermé et l'état proximal ouvert.

La seconde pièce 16 est de forme générale tubulaire et s'étend en direction axiale sur une longueur plus grande que celle de la première pièce 15, en particulier au moins égale à celle de la première pièce augmentée de la course C, et plus spécialement sur une longueur proche de celle de la première pièce 15 augmentée de la course C, comme dans la réalisation représentée.

La seconde pièce 16 comporte un corps 35 et, à sa partie extrême proximale, la tête 14, disposée transversalement. Une pièce formant entretoise 47 relie le corps 35 à la tête 14 qui a une position fixe par rapport au corps 35. Le terme « entretoise » n'est pas limitatif en soi et se réfère au fait que la pièce 47 est un organe support suffisamment rigide s'étendant en direction axiale apte à maintenir la tête 14 de façon fixe par rapport au corps 35. Cette pièce formant entretoise 47 est agencée pour ménager une ouverture de passage latérale 48 permettant le passage à travers du contenu du récipient 2.

La pièce formant entretoise 47 peut se présenter sous différentes formes équivalentes : en plusieurs parties telles que des tiges espacées les unes des autres, en une seule partie telle qu'un manchon ajouré, par exemple. L'ouverture de passage latérale 48 est, comme dans la réalisation représentée, en plusieurs parties et elle est définie par la forme donnée à la pièce formant entretoise 47.

La longueur axiale de la pièce formant entretoise 47 est adaptée pour permettre à a partie proximale active 9 d'être au contact du contenu du récipient 2.

Au-delà du corps 35, en direction proximale et entre la limite proximale du corps 35 et la tête 14 est ménagé un espace 18a. Cet espace 18a a une paroi proximale formée par la tête adjacente 14, une limite distale 27 - qui est la limite proximale du corps 35 - et l'ouverture de passage latérale 48.

Le corps de la seconde pièce 16 comporte à sa partie extrême distale 26, la partie d'actionnement 17 qui, une fois actionnée, par exemple manuellement, assure le coulissement axial de la seconde pièce 16 dans la première pièce 15.

Le corps de la seconde pièce 16 forme un support de l'accessoire 3 de sorte que sa partie proximale active 9 soit située dans l'espace 18a. Dans la réalisation représentée, l'accessoire 3 est fixé d'origine à la seconde pièce 16, en particulier par surmoulage de la partie distale 10 de l'accessoire 3.

La tête 14 a deux fonctions :
- Dans l'état distal fermé, la tête 14 ferme de façon étanche l'ouverture 7 et le dispositif 1 de raccordement du côté proximal, en formant une barrière interdisant l'accès à l'intérieur 4 du récipient 2 et la communication entre l'accessoire 3 et l'intérieur 4 du récipient 2.
- Dans l'état proximal ouvert, la tête 14 contribue à la protection de la partie proximale active 9 surtout si elle est introduite à proprement parler dans l'intérieur 4 du récipient 2, en ayant franchi l'ouverture 7.

La tête 14 se présente sous la forme générale d'une paroi transversale, pleine. Elle est de forme et de dimension latérale périphérique correspondant à celles de l'intrados de la première pièce 15, de sorte à être apte à être introduite axialement dans la première pièce 15 dans la direction proximale, depuis son ouverture d'extrémité distale 31 et ensuite à être déplacée à coulissement axial dans la première pièce 15 dans la direction proximale, depuis son ouverture d'extrémité distale 31, jusqu'à son ouverture d'extrémité proximale 30.

La tête 14 est garnie sur son chant latéral d'un organe d'étanchéité latéral de tête 28, tel qu'un joint torique de tête, en silicone ou autre matériau équivalent, coopérant de façon étanche, à l'état distal fermé, avec l'intrados de la paroi tubulaire 14 de la première pièce vers la partie extrême proximale 32. La tête 14 avec son joint 28 est donc ajustée pour venir coopérer avec étanchéité dans l'ouverture d'extrémité proximale 30 ou dans la partie extrême proximale 32 de la première pièce 15.

Avec les dispositions constructives indiquées, la seconde pièce 16 est apte à être pré assemblée à la première pièce 15 moyennant un coulissement axial dans la direction proximale. Ainsi, la première pièce 15 (plus généralement le premier moyen) et la seconde pièce 16 (plus généralement le second moyen) ainsi pré assemblées forment un tout structurel apte à être assemblé à ladite partie complémentaire d'assemblage 19 du récipient 2, par un coulissement axial dans la direction proximale.

La seconde pièce 16 et la première pièce 15 sont mutuellement agencées de sorte à coopérer, la seconde pièce 16 étant dans la première pièce 15 et pouvant au moins coulisser par rapport à elle. Un tel agencement peut résulter d'un montage à coulissement. Il peut aussi résulter d'un montage à coulissement et pivotement, par la coopération d'organes complémentaires prévus sur la première pièce 15 et la seconde pièce 16, tels qu'un moyen de liaison cinématique de type hélicoïdal, tel qu'un pivotement axial relatif imprimé à la seconde pièce 16 entraîne son coulissement axial le long de la première pièce 15. Un tel moyen de liaison cinématique de type hélicoïdal peut, en particulier, comporter un ergot latéral d'une pièce coopérant avec une gorge hélicoïdale de l'autre pièce.

La seconde pièce 16 est montée axialement dans la première pièce 15 avec interposition, ici, de deux organes d'étanchéité latéraux 39a, 39b, sous la forme d'un ou plusieurs joints portés par l'extrados de la seconde pièce 16. L'organe d'étanchéité distal 39a et l'organe d'étanchéité proximal 39b sont écartés l'un de l'autre en direction axiale.

L'écartement en direction axiale entre l'organe d'étanchéité distal 39a et l'organe d'étanchéité proximal 39b est plus grand, en particulier légèrement plus grand, que l'écartement en direction axiale entre l'organe d'étanchéité proximal 39b et l'organe d'étanchéité de tête 28, ceux-ci pouvant être écartés en direction axiale d'un écartement égal ou voisin de la course C.

Le joint d'étanchéité de tête 18 est un joint d'étanchéité au produit biopharmaceutique fluide.

Le joint d'étanchéité proximal 39b est un joint d'étanchéité au produit biopharmaceutique fluide et aux éventuels contaminants d'origine extérieure.

Le joint d'étanchéité distal 39a est un joint d'étanchéité aux éventuels contaminants d'origine extérieure.

Toutes les dispositions constructives précédentes permettent de combiner à la fois un bon guidage par une portée suffisante, un bon coulissement, une bonne étanchéité et une prévention des contaminations aux éventuels contaminants d'origine extérieure à l'occasion d'un coulissement de va et vient de la seconde pièce 16. En outre, le premier moyen (première pièce) 15 et le second moyen (seconde pièce 16) peuvent être pré assemblés de sorte à former un tout structurel à l'état distal fermé. Et ce tout structurel peut inclure l'accessoire 3 fixé à la seconde pièce 16, en particulier par surmoulage de la partie distale 10 et un liquide de calibration de l'accessoire 3 emplissant la chambre 18. Par ailleurs, les dispositions constructives décrites ci-avant permettent d'activer plusieurs fois la valve selon la séquence état fermé → état ouvert → état fermé → état ouvert - ce qui peut notamment s'avérer utile pour réaliser un recalibrage de l'accessoire 3 - tout en garantissant la non contamination du contenu biopharmaceutique du récipient 2 par des microorganismes due au fait de la manipulation du dispositif de raccordement 1.

Le dispositif 1 de raccordement comprend la chambre 18, laquelle inclut l'espace 18a. A l'état distal fermé, la chambre 18 est fermée latéralement par la paroi tubulaire 24 de la première pièce 15 qui occulte l'ouverture de passage latérale 48, l'étanchéité étant assurée. Dans cette situation, la chambre 18 est apte à contenir, de façon étanche, un liquide de conservation, réglage, contrôle, ou calibration de l'accessoire 3. A l'état proximal ouvert, la chambre 18 est dégagée de la première pièce 15 et ouverte par l'ouverture de passage latérale 48. Elle est alors apte à être en communication avec l'intérieur 4 par l'ouverture de passage latérale 48 et ainsi, il en est de même de la partie proximale active 9 de l'accessoire 3.

Selon les applications et les besoins, la chambre 18 peut être stérile ou non stérile humide ou sèche, en fonction de l'accessoire 3. Par exemple, une sonde pH requiert une ambiance humide. Cette ambiance humide est obtenue par la présence, dans la chambre 18 d'un liquide *ad hoc*, injecté au moment du stockage et désigné pour cette raison « liquide de stockage ».

Dans le cas où, au contraire, l'accessoire 3 requiert non pas une ambiance humide mais une ambiance sèche, comme dans le cas où l'accessoire 3 est un tube, il n'est pas prévu un tel liquide de stockage.

Une fonction de la chambre 18, dans le cas où l'accessoire 3 est une sonde 3, est de procéder à une calibration de celle-ci. A cet effet, on met dans la chambre 18 un liquide de calibration.

L'ensemble (1 + 3) pour la mesure ou le contrôle d'un paramètre d'un liquide se trouvant à l'intérieur 4 du récipient 2 comprend le dispositif 1 de raccordement et l'accessoire 3, et, le cas échéant, le liquide de calibration de l'accessoire 3 emplissant la chambre 18 fermée, et, le cas échéant, un organe de raccordement extérieur 5 et un ou plusieurs organes 5a, comme précédemment décrits. Un tel ensemble 1 + 3 est prêt à être monté sur le récipient 2.

L'ensemble (1 + 2 + 3) à récipient pour application biopharmaceutique avec mesure ou contrôle d'un paramètre d'un liquide se trouvant à l'intérieur 4 du récipient 2 comprend le récipient 2 avec sa paroi 6 pourvue d'une ouverture 7 et, fixée de façon rigide et étanche, la partie complémentaire d'assemblage 19 qui coopère avec le dispositif 1 de raccordement.

Un ensemble 1 + 3 pour la mesure ou le contrôle peut être assemblé avant que d'être monté sur la partie complémentaire 19 du récipient 2, raison pour laquelle on parle de pré assemblage.

Le procédé de pré assemblage est caractérisé par la succession des étapes suivantes, une fois que l'on dispose de moyens aptes à constituer un dispositif 1 de raccordement et d'un accessoire de mesure ou de contrôle 3 :
▪ on agence le second moyen 16 du dispositif 1 de raccordement et l'accessoire 3, pour que le second moyen 16 supporte l'accessoire 3 de façon que sa partie active 9 soit située dans l'espace 18a,
▪ puis, on fait coulisser axialement le second moyen 16 dans le premier moyen 15 depuis son ouverture d'extrémité distale 31 en direction proximale, jusqu'à l'état distal fermé.

Selon une réalisation, à l'état distal fermé, la chambre 18 fermée est emplie d'un liquide de calibration de l'accessoire 3.

Compte tenu des caractéristiques constructives du dispositif 1 de raccordement, le pré assemblage qui vient d'être décrit peut être réalisé sans la nécessité d'être confiné dans une salle blanche.

Pour monter un ensemble 1 + 2 + 3 à récipient, on procède à la succession d'étapes suivantes, une fois que l'on dispose d'un récipient 2 vide de contenu et pourvu de la partie complémentaire d'assemblage 19 et d'un ensemble 1 + 2 pour la mesure ou le contrôle: on fait coulisser axialement l'ensemble pour la mesure ou le contrôle 1 + 2 dans la partie complémentaire d'assemblage 19 depuis son ouverture d'extrémité distale 21 en direction proximale, jusqu'à l'état distal fermé. Pour les applications biopharmaceutiques, il est prévu une étape ultérieure de stérilisation de la totalité de l'ensemble 1 + 2 + 3 à récipient, par exemple par rayon gamma, cette réalisation n'étant toutefois pas limitative.

Pour mettre en oeuvre un tel ensemble 1 + 3 pour la mesure ou le contrôle faisant partie d'un ensemble 1 + 2 +3 à récipient, on procède à la succession des étapes suivantes :
▪ la chambre 18 étant vide, on fait coulisser axialement le second moyen 16 du dispositif 1 de raccordement de l'état distal fermé à l'état proximal ouvert, dans la direction proximale,
▪ puis, on met en oeuvre l'accessoire 3.

Selon une réalisation typique lorsque l'accessoire 3 est une sonde telle qu'une sonde à pH, le procédé comporte au moins une opération supplémentaire de conservation, de réglage, de contrôle, ou, typiquement, de calibration de l'accessoire 3. Une telle opération est une opération initiale avant que le récipient 2 soit empli de son contenu, une opération finale, après que le récipient 2 a été vidangé de son contenu, et le cas échéant, une opération après que le récipient 2 a été empli de son contenu et avant qu'il a été vidangé de son contenu. Une telle opération est réalisée alors que l'ensemble 1 + 3 pour la mesure ou le contrôle et son dispositif 1 de raccordement sont à l'état distal fermé et que la chambre 18 est fermée de façon étanche.

## Revendications

1. Dispositif (1) de raccordement étanche à un récipient (2) d'un accessoire (3) dont la partie proximale active (9) est destinée à être mise en communication avec l'intérieur (4) du récipient (2) par une ouverture (7) du récipient (2), le dispositif (1) présentant un axe (1 a) et comprenant :
▪ un premier moyen (15), de forme générale tubulaire avec une ouverture d'extrémité proximale (30) et une ouverture d'extrémité distale (31), agencé de sorte à assurer la fixation du dispositif (1) au récipient (2), à constituer une voie de passage entre l'intérieur (4) et l'extérieur (8) du récipient (2), à former un support de guidage à coulissement axial d'un second moyen (16) entre un état distal fermé et un état proximal ouvert, sur une course axiale C, à contribuer à former une chambre (18),
▪ un second moyen (16), de forme générale allongée, monté axialement dans le premier moyen (15), avec interposition d'un organe d'étanchéité latéral (39a, 39b), de sorte à pouvoir être déplacé à coulissement axial sur la course C entre l'état distal fermé et l'état proximal ouvert, agencé de sorte à comporter une tête (14), transversale, à sa partie extrême proximale qui est apte, à l'état distal fermé, à fermer de façon étanche l'ouverture (7), à comporter un espace (18a) ayant une paroi proximale formée par la tête (14) adjacente, une limite distale (27), et une ouverture de passage latérale (48), à comporter à sa partie extrême distale une partie d'actionnement à coulissement axial (17), et à former un support de l'accessoire (3) de sorte que sa partie active (9) soit située dans ledit espace (18a), à contribuer à former une chambre (18),
▪ une chambre (18), incluant ledit espace (18a) et qui, à l'état distal fermé, est fermée latéralement par le premier moyen (15) et, le cas échéant, est apte à contenir, de façon étanche, un liquide de conservation, réglage, contrôle ou calibration de l'accessoire (3), et qui, à l'état proximal ouvert, est dégagée du premier moyen (15) et, ainsi est apte à être en communication avec l'intérieur (4) par l'ouverture de passage latérale (48),
**caractérisé par le fait que**
le premier moyen (15) comporte une partie extrême proximale (32) d'assemblage fixe par coulissement, agencée de sorte à pouvoir être assemblée de façon fixe et étanche, avec interposition d'un organe d'étanchéité latéral (22), sur une partie complémentaire (19) d'assemblage fixe du récipient (2), moyennant au moins un coulissement axial dans la direction proximale,
▪ la tête transversale (14) est de forme et de dimension latérale périphérique correspondant à celles de l'intrados du premier moyen (15), de sorte à être apte à coulisser axialement dans le premier moyen (15) dans la direction proximale, depuis son ouverture distale (31) jusqu'à sa partie extrême proximale (32), la tête (14) étant garnie d'un organe d'étanchéité latéral de tête (28), tel qu'un joint de tête,
▪ le second moyen (16) est apte à être pré assemblé au premier moyen (15) moyennant un coulissement axial dans la direction proximale, le premier moyen (15) et le second moyen (16) ainsi pré assemblés formant un tout structurel apte à être assemblé à ladite partie complémentaire d'assemblage (19) par un coulissement axial dans la direction proximale.

2. Dispositif (1) de raccordement selon la revendication 1, **caractérisé par le fait qu'**à l'état distal fermé, la tête (14) est située à ou adjacente à l'ouverture proximale (30) du premier moyen (15), et/ou la partie d'actionnement (17) est située écartée de la partie extrême distale (33) du premier moyen (15), dans la direction distale, d'une distance au moins égale à la course C et/ou **caractérisé par le fait qu'**à l'état proximal ouvert, la tête (14) est située écartée de l'ouverture proximale (30) du premier moyen (15), dans la direction proximale, d'une distance au moins égale à la course C, et/ou la partie d'actionnement (17) est située à, ou adjacente de, ou proche de, la partie extrême distale (33) du premier moyen (15), en particulier hors du premier moyen (15).

3. Dispositif (1) de raccordement selon l'une quelconque des revendications 1 et 2, **caractérisé par le fait que** le premier moyen (15) comprend, en particulier est constitué par, une première pièce unique (15) et/ou le second moyen (16) comprend, en particulier est constitué par, une seconde pièce unique (16), avec organes d'étanchéité latéraux rapportés (39a, 39b) et/ou **caractérisé par le fait que** le premier moyen (15) comporte une première pièce (15) de forme générale tubulaire s'étendant en direction axiale sur une longueur plus grande que la course C, en particulier substantiellement plus grande que la course C, dont l'extrados est de forme et de dimension latérale périphérique correspondant à celles de l'intrados de ladite partie complémentaire d'assemblage, de sorte à être apte à être montée axialement dans ladite partie complémentaire d'assemblage (19), ladite première pièce (15) ayant un rebord radial latéral vers l'extérieur (25) apte à coopérer en blocage axial dans la direction proximale avec un bord (21) de ladite partie complémentaire d'assemblage (19) et/ou un organe d'étanchéité (22) étant interposé entre l'extrados de ladite première pièce (15) et l'intrados de ladite partie complémentaire d'assemblage (19), en particulier un joint (22) porté par l'extrados de ladite première pièce (15).

4. Dispositif (1) de raccordement selon l'une quelconque des revendications 1 à 3, **caractérisé par le fait que**
le second moyen (16) comporte une seconde pièce (16) de forme générale tubulaire s'étendant en direction axiale sur une longueur plus grande que celle du premier moyen (15), en particulier au moins égale à celle du premier moyen (15) augmentée de la course C, et plus spécialement sur une longueur proche de celle du premier moyen (15) augmentée de la course C, et/ou une seconde pièce (16) pourvue à sa partie extrême distale (26) d'un rebord radial latéral vers l'extérieur (17) apte à coopérer en blocage axial dans la direction proximale avec le bord de la partie extrême distale (33) du premier moyen (15), formant le cas échéant partie d'actionnement (17) et/ou
le second moyen (16) est monté axialement dans le premier moyen (15) avec interposition d'un ou plusieurs organes d'étanchéité latéraux (39a, 39b) sous la forme d'un ou plusieurs joints portés par l'extrados du second moyen (16), et/ou **caractérisé par** un organe d'étanchéité distal (39a) et un organe d'étanchéité proximal (39b) écartés l'un de l'autre en direction axiale, et/ou **caractérisé par le fait que** l'écartement en direction axiale entre l'organe d'étanchéité distal (39a) et l'organe d'étanchéité proximal (39b) est plus grand, en particulier légèrement plus grand, que l'écartement en direction axiale entre l'organe d'étanchéité proximal (39b) et l'organe d'étanchéité de tête (28), et/ou **caractérisé par le fait que** l'écartement en direction axiale entre l'organe d'étanchéité proximal (39b) et l'organe d'étanchéité de tête (28) est égal ou voisin de la course C.

5. Dispositif (1) de raccordement selon l'une quelconque des revendications 1 à 4, **caractérisé par le fait qu'**est ménagée dans le second moyen (16) une conduite (11), tel qu'un perçage, de liquide, s'étendant axialement, associée fonctionnellement à l'accessoire (3) en particulier en vue de sa conservation, son réglage, contrôle ou calibration, ayant une ouverture d'extrémité proximale (12) débouchant dans ledit espace (18a), respectivement dans la dite chambre (18), et une ouverture d'extrémité distale (13) débouchant vers la partie extrême distale (26) du second moyen (16), agencée de sorte que la conduite soit apte à être prolongée en direction distale hors du second moyen (16), par un organe de raccordement extérieur (5), tel que comprenant au moins un tube, et/ou **caractérisé par le fait qu'**il comporte au moins deux conduites (11) respectivement d'amenée et de vidange.

6. Dispositif (1) de raccordement selon l'une quelconque des revendications 1 à 5, **caractérisé par le fait que** le premier moyen (15) et le second moyen (16) sont pré assemblés de sorte à former un tout structurel à l'état distal fermé, et/ou **caractérisé par le fait que**, d'origine, l'accessoire (3) est fixé au second moyen (16), en particulier par surmoulage de la partie distale (10) de l'accessoire (3), et/ou la chambre (18), fermée, est emplie d'un liquide de calibration de l'accessoire (3).

7. Ensemble (1 + 3) pour la mesure ou le contrôle d'un paramètre d'un liquide se trouvant à l'intérieur (4) d'un récipient (2) au moyen d'un accessoire de mesure ou de contrôle (3) dont la partie proximale active (9) est destinée à être mise en communication avec l'intérieur (4) du récipient (2) par une ouverture (7) du récipient (2), ledit ensemble (1 + 3) pour la mesure ou le contrôle comprenant ledit accessoire (3), un dispositif (1) de raccordement au récipient (2) dudit accessoire (3) selon l'une quelconque des revendications 1 à 6, et, le cas échéant, un liquide de calibration de l'accessoire (3) emplissant la chambre (18) fermée dudit dispositif (1) de raccordement à l'état distal fermé, ledit ensemble pour la mesure ou le contrôle (1 + 3) étant prêt à être monté sur un récipient (2), et/ou le dispositif (1) de raccordement est du type dans lequel est ménagé dans le second moyen (16) une ou plusieurs conduites (11), **caractérisé par le fait qu'**il comporte également, associé à la conduite (11) ou à chacune des conduites (11), par une liaison fixe et communicante, l'organe de raccordement extérieur (5), tel que comprenant au moins un tube et/ou, soit comporte
un ou plusieurs organes (5a) de connexion, d'arrêt, de circulation, de filtration, de mesure, de réception pour l'amenée ou la collecte du liquide destiné à passer dans ledit organe de raccordement extérieur et ladite conduite associée, soit comporte
un ou plusieurs organes (5a) d'échantillonnage apte à récupérer un échantillon du contenu du récipient (2) emprisonné dans la chambre après le passage du dispositif de raccordement (1) de l'état ouvert à l'état fermé.

8. Ensemble (1 + 3) pour la mesure ou le contrôle selon la revendication 7, **caractérisé par le fait que** l'accessoire (3) est une sonde de mesure du pH du contenu se trouvant à l'intérieur (4) du récipient (2).

9. Ensemble (1 + 2 + 3) à récipient pour application biopharmaceutique avec mesure ou contrôle d'un paramètre d'un liquide se trouvant à l'intérieur (4) du récipient (2), comprenant un récipient (2) ayant une paroi (6) pourvue d'une ouverture (7) et, fixée de façon rigide et étanche à ladite paroi (6) autour de l'ouverture (7), une partie complémentaire d'assemblage (19) fixe et étanche apte à coopérer avec la partie d'extrémité proximale (30) d'assemblage fixe du premier moyen (15) d'un dispositif (1) de raccordement selon l'une quelconque des revendications 1 à 6 ou d'un ensemble pour la mesure ou le contrôle (1 + 3) selon l'une quelconque des revendications 7 et 8, et/ou **caractérisé par le fait que** partie complémentaire d'assemblage (19) fixe et étanche a une forme générale tubulaire ayant une paroi latérale (19a) avec une extrémité proximale avec ouverture (20) et une extrémité distale avec ouverture (21), et une collerette (23) d'extrémité proximale apte à être solidarisée de manière fixe et étanche à la paroi (6) du récipient (2) autour de son ouverture (7).

10. Ensemble (1 + 2 + 3) à récipient selon la revendication 9, **caractérisé par le fait que** le récipient (2) est soit rigide et réutilisable soit souple et à usage unique, tel qu'une poche dite 2D ou 3D, et/ou dans lequel le récipient (2) est souple, **caractérisé par le fait qu'**il est prévu des moyens rigides de maintien extérieur du récipient (2) pourvus d'un passage pour le dispositif (1) de raccordement ou l'ensemble pour la mesure ou le contrôle.

11. Procédé de pré assemblage d'un ensemble (1 + 3) pour la mesure ou le contrôle d'un paramètre selon l'une quelconque des revendications 7 et 8, en vue de son montage ultérieur sur un récipient (2), **caractérisé par** la succession des étapes suivantes :
▪ on dispose de moyens aptes à constituer un dispositif (1) de raccordement selon l'une quelconque des revendications 1 à 6,
▪ on dispose d'un accessoire de mesure ou de contrôle (3),
▪ on agence le second moyen (16) du dispositif (1) de raccordement et l'accessoire (3), pour que le second moyen (16) supporte l'accessoire (3) de façon que sa partie active (9) soit située dans ledit espace (18a) ménagé par le second moyen (16),
▪ on fait coulisser axialement le second moyen (16) dans le premier moyen (15) depuis son ouverture d'extrémité distale (31) en direction proximale, jusqu'à l'état distal fermé, et/ou **caractérisé par le fait que**, à l'état distal fermé, la chambre (18) fermée est emplie d'un liquide de conservation et/ou calibration de l'accessoire (3).

12. Procédé de pré assemblage selon la revendication 11, **caractérisé par le fait qu'**il est réalisé sans la nécessité d'être confiné dans une salle blanche.

13. Procédé de montage d'un ensemble (1 + 2 + 3) à récipient selon la revendication 8, **caractérisé par** la succession des étapes suivantes :
▪ on dispose d'un récipient (2) vide de contenu, pourvu, autour d'une ouverture (7) d'une partie complémentaire d'assemblage (19) avec un dispositif (1) de raccordement,
▪ on dispose d'un ensemble (1 + 3) pour la mesure ou le contrôle d'un paramètre selon la revendication 7,
▪ on fait coulisser axialement l'ensemble (1 + 3) pour la mesure ou le contrôle d'un paramètre dans ladite partie complémentaire d'assemblage (19) depuis son ouverture d'extrémité distale (31) en direction proximale, jusqu'à l'état distal fermé, et/ou **caractérisé par** une étape ultérieure de stérilisation de l'ensemble (1 + 2 + 3) à récipient.

14. Procédé de mise en oeuvre d'un ensemble (1 + 3) pour la mesure ou le contrôle d'un paramètre faisant partie d'un ensemble (1 + 2 + 3) à récipient monté selon l'une quelconque des revendications 8 à 10, **caractérisé par** la succession des étapes suivantes :
▪ la chambre (18) étant vide, on fait coulisser axialement le second moyen (16) du dispositif (1) de raccordement de l'état distal fermé à l'état proximal ouvert, dans la direction proximale,
▪ on met en oeuvre l'accessoire (3).

15. Procédé de mise en oeuvre selon la revendication 14, **caractérisé par le fait qu'**il comporte au moins une opération supplémentaire de conservation, de réglage, de contrôle ou de calibration de l'accessoire (3), en particulier une opération initiale avant que le récipient (2) soit empli de son contenu, une opération finale, après que le récipient (2) a été vidangé de son contenu, et le cas échéant, une opération après que le récipient (2) a été empli de son contenu et avant qu'il a été vidangé de son contenu, alors que le l'ensemble pour la mesure ou le contrôle d'un paramètre et son dispositif (1) de raccordement sont à l'état distal fermé et que la chambre (18) est fermée de façon étanche.

## Patentansprüche

1. Vorrichtung (1) zur dichten Verbindung eines Zubehörteils (3) mit einem Behälter (2), dessen aktiver proximaler Teil (9) dazu bestimmt ist, mit dem Inneren (4) des Behälters (2) durch eine Öffnung (7) des Behälters (2) verbunden zu werden, wobei die Vorrichtung (1) eine Achse (1 a) aufweist und umfasst:
- ein erstes Mittel (15) von allgemeiner röhrenförmiger Form mit einer proximalen Endöffnung (30) und einer distalen Endöffnung (31), das derart angeordnet ist, dass es die Befestigung der Vorrichtung (1) am Behälter (2) gewährleistet, einen Durchgangsweg zwischen dem Inneren (4) und dem Äußeren (8) des Behälters (2) darstellt, eine Stütze zur axialen Gleitführung eines zweiten Mittels (16) zwischen einem geschlossenen distalen Zustand und einem offenen proximalen Zustand auf einer axialen Strecke C bildet, und dazu beiträgt, eine Kammer (18) zu bilden,
- ein zweites Mittel (16) von allgemeiner länglicher Form, das axial in dem ersten Mittel (15) montiert ist, wobei ein seitliches Abdichtungselement (39a, 39b) dazwischen angeordnet ist, so dass es axial gleitend auf der Strecke C zwischen dem geschlossenen distalen Zustand und dem offenen proximalen Zustand verschoben werden kann, wobei es derart angeordnet ist, dass es einen quer verlaufenden Kopf (14) an seinem proximalen äußersten Teil umfasst, der geeignet ist, in dem geschlossenen distalen Zustand die Öffnung (7) dicht zu verschließen, einen Raum (18a) mit einer proximalen Wand, die von dem angrenzenden Kopf (14) einer distalen Grenze (27) und einer seitlichen Durchgangsöffnung (48) gebildet ist, zu umfassen, an seinem distalen äußersten Teil einen axial gleitenden Betätigungsteil (17) zu umfassen und eine Stütze für den Zubehörteil (3) zu bilden, so dass sich sein aktiver Teil (9) in dem Raum (18a) befindet, und dazu beizutragen, eine Kammer (18) zu bilden,
- eine Kammer (18), die den Raum (18a) einschließt, und die im geschlossenen distalen Zustand seitlich durch das erste Mittel (15) verschlossen ist, und die gegebenenfalls geeignet ist, auf dichte Weise eine Flüssigkeit zur Konservierung, Regelung, Kontrolle oder Eichung des Zubehörteils (3) zu enthalten, und die im offenen proximalen Zustand frei von dem ersten Mittel (15) und somit geeignet ist, mit dem Inneren (4) durch die seitliche Durchgangsöffnung (48) in Verbindung zu sein,
**dadurch gekennzeichnet, dass**
- das erste Mittel (15) einen proximalen äußersten Teil (32) zur festen Verbindung durch Gleiten umfasst, der derart angeordnet ist, dass er auf feste und dichte Weise unter Zwischenschaltung eines seitlichen Abdichtungselements (22) mit einem festen komplementären Verbindungsteil (19) des Behälters (2) durch mindestens ein axiales Gleiten in proximale Richtung verbunden werden kann,
- der quer verlaufende Kopf (14) eine periphere seitliche Form und Dimension aufweist, die jenen der Innenseite des ersten Mittels (15) entsprechen, um geeignet zu sein, axial in dem ersten Mittel (15) in proximale Richtung von seiner distalen Öffnung (31) bis zu seinem proximalen äußersten Teil (32) zu gleiten, wobei der Kopf (14) mit einem seitlichen Kopfabdichtungselement (28), wie einer Kopfdichtung, versehen ist,
- das zweite Mittel (16) geeignet ist, mit dem ersten Mittel (15) durch ein axiales Gleiten in proximale Richtung vorverbunden zu werden, wobei die auf diese Weise vorverbundenen ersten Mittel (15) und zweiten Mittel (16) eine strukturelle Einheit bilden, die geeignet ist, mit dem komplementären Verbindungsteil (19) durch ein axiales Gleiten in proximale Richtung verbunden zu werden.

2. Verbindungsvorrichtung (1) nach Anspruch 1, **dadurch gekennzeichnet, dass** sich der Kopf (14) im geschlossenen distalen Zustand an der proximalen Öffnung (30) des ersten Mittels (15) angeordnet oder anliegend befindet, und/oder sich der Betätigungsteil (17) von dem distalen äußersten Teil (33) des ersten Mittels (15) in distaler Richtung um einem Abstand mindestens gleich der Strecke C entfernt befindet, und/oder **dadurch gekennzeichnet, dass** sich der Kopf (14) im offenen proximalen Zustand von der proximalen Öffnung (30) des ersten Mittels (15) in proximaler Richtung um einen Abstand mindestens gleich der Strecke C entfernt befindet, und/oder sich der Betätigungsteil (17) an dem distalen äußersten Teil (33) des ersten Mittels (15) angeordnet, anliegend oder nahe, insbesondere außerhalb des ersten Mittels (15) befindet.

3. Verbindungsvorrichtung (1) nach einem der Ansprüche 1 und 2, **dadurch gekennzeichnet, dass** das erste Mittel (15) einen ersten einzigen Teil (15) umfasst, insbesondere von diesem gebildet ist, und/oder das zweite Mittel (16) einen zweiten einzigen Teil (16) umfasst, insbesondere von diesem gebildet ist, mit aufgesetzten seitlichen Abdichtungselementen (39a, 39b), und/oder **dadurch gekennzeichnet, dass** das erste Mittel (15) einen ersten Teil (15) von allgemeiner röhrenförmiger Form umfasst, der sich in Axialrichtung auf einer größeren Länge als die Strecke C, insbesondere wesentlich größer als die Strecke C, erstreckt, dessen Außenseite von peripherer seitlicher Form und Dimension ist, die jenen der Innenseite des komplementären Verbindungsteils entsprechen, so dass er geeignet ist, axial in dem komplementären Verbindungsteil (19) montiert zu werden, wobei der erste Teil (15) einen seitlichen radialen Rand nach außen (25) hat, der geeignet ist, in axialer Feststellung in proximale Richtung mit einem Rand (21) des komplementären Verbindungsteils (19) zusammenzuwirken, und/oder wobei ein Abdichtungselement (22) zwischen der Außenseite des ersten Teils (15) und der Innenseite des komplementären Verbindungsteils (19) angeordnet ist, insbesondere eine Dichtung (22), die von der Außenseite des ersten Teils (15) getragen wird.

4. Verbindungsvorrichtung (1) nach einem der Ansprüche 1 bis 3, **dadurch gekennzeichnet, dass**
das zweite Mittel (16) einen zweiten Teil (16) von allgemeiner röhrenförmiger Form umfasst, der sich in Axialrichtung auf einer größeren Länge als jene des ersten Mittels (15), insbesondere zumindest gleich jener des ersten Mittels (15), verlängert um die Strecke C, und noch spezieller auf einer ähnlichen Länge wie jene des ersten Mittels (15), verlängert um die Strecke C, erstreckt, und/oder einen zweiten Teil (16), der an seinem distalen äußersten Teil (26) mit einem seitlichen radialen Rand nach außen (17) versehen ist, der geeignet ist, in axialer Feststellung in proximale Richtung mit dem Rand des distalen äußersten Teils (33) des ersten Mittels (15) zusammenzuwirken, wobei er gegebenenfalls den Betätigungsteil (17) bildet,
und/oder
das zweite Mittel (16) axial in dem ersten Mittel (15) montiert ist, unter Zwischenschaltung eines oder mehrerer seitlicher Abdichtungselemente (39a, 39b) in Form einer oder mehrerer Dichtungen, die von der Außenseite des zweiten Mittels (16) getragen werden, und/oder **gekennzeichnet durch** ein distales Abdichtungselement (39a) und ein proximales Abdichtungselement (39b), die voneinander in Axialrichtung entfernt sind,
und/oder **dadurch** gekennzeichnet, dass die Entfernung in Axialrichtung zwischen dem distalen Abdichtungselement (39a) und dem proximalen Abdichtungselement (39b) größer, insbesondere etwas größer, als die Entfernung in Axialrichtung zwischen dem proximalen Abdichtungselement (39b) und dem Kopfabdichtungselement (28) ist, und/oder **dadurch** gekennzeichnet, dass die Entfernung in Axialrichtung zwischen dem proximalen Abdichtungselement (39b) und dem Kopfabdichtungselement (28) gleich oder ähnlich der Strecke C ist.

5. Verbindungsvorrichtung (1) nach einem der Ansprüche 1 bis 4, **dadurch gekennzeichnet, dass** in dem zweiten Mittel (16) eine Leitung (11), wie eine Bohrung, für Flüssigkeit ausgenommen ist, die sich axial erstreckt, funktionell mit dem Zubehörteil (3) insbesondere im Hinblick auf ihre Konservierung, Regelung, Kontrolle oder Eichung verbunden ist, die eine proximale Endöffnung (12), die in den Raum (18a) jeweils in der Kammer (18) mündet, und eine distale Endöffnung (13) besitzt, die in den distalen Endteil (26) des zweiten Mittels (16) mündet, derart angeordnet, dass die Leitung geeignet ist, in distale Richtung außerhalb des zweiten Mittels (16) durch ein äußeres Verbindungselement (5), wie ein mindestens ein Rohr umfassendes, verlängert zu werden, und/oder **dadurch gekennzeichnet, dass** sie mindestens zwei Leitungen (11) für die Zuleitung bzw. die Ableitung umfasst.

6. Verbindungsvorrichtung (1) nach einem der Ansprüche 1 bis 5, **dadurch gekennzeichnet, dass** das erste Mittel (15) und das zweite Mittel (16) vorverbunden sind, um eine strukturelle Einheit im geschlossenen distalen Zustand zu bilden, und/oder **dadurch gekennzeichnet, dass** ursprünglich der Zubehörteil (3) an dem zweiten Mittel (16) insbesondere durch Aufformen des distalen Teils (10) des Zubehörteils (3) befestigt ist, und/oder die Kammer (18), die geschlossen ist, mit einer Flüssigkeit zur Eichung des Zubehörteils (3) gefüllt ist.

7. Einheit (1 + 3) zur Messung oder Kontrolle eines Parameters einer Flüssigkeit, die sich im Inneren (4) eines Behälters (2) befindet, mit Hilfe eines Mess- oder Kontrollzubehörteils (3), dessen aktiver proximaler Teil (9) dazu bestimmt ist, mit dem Inneren (4) des Behälters (2) durch eine Öffnung (7) des Behälters (2) verbunden zu werden, wobei die Einheit (1 + 3) zur Messung oder Kontrolle den Zubehörteil (3), eine Vorrichtung (1) zur Verbindung des Zubehörteils (3) mit dem Behälter (2) nach einem der Ansprüche 1 bis 6 und gegebenenfalls eine Flüssigkeit zur Eichung des Zubehörteils (3) umfasst, mit der die geschlossene Kammer (18) der Verbindungsvorrichtung (1) im geschlossenen distalen Zustand gefüllt ist, wobei die Einheit zur Messung oder Kontrolle (1 + 3) bereit ist, auf einem Behälter (2) montiert zu werden, und/oder wobei die Verbindungsvorrichtung (1) von dem Typ ist, bei dem in dem zweiten Mittel (16) eine oder mehrere Leitungen (11) ausgenommen sind, **dadurch gekennzeichnet, dass** sie auch, verbunden mit der Leitung (11) oder jeder der Leitungen (11) durch eine feste und durchgehende Verbindung das äußere Verbindungselement (5), wie ein zumindest ein Rohr umfassendes, umfasst, und/oder entweder
ein oder mehrere Elemente (5a) zur Verbindung, zum Anhalten, zur Zirkulation, Filterung, Messung, Aufnahme für die Zuleitung oder das Sammeln der Flüssigkeit umfasst, die dazu bestimmt ist, das äußere Verbindungselement und die zugehörige Leitung zu durchströmen, oder
ein oder mehrere Elemente (5a) zur Probenahme umfasst, die geeignet sind, eine Probe des Inhalts des Behälters (2), der in der Kammer nach dem Übergang der Verbindungsvorrichtung (1) vom offenen Zustand in den geschlossenen Zustand eingeschlossen ist, zu entnehmen.

8. Einheit (1 + 3) zur Messung oder Kontrolle nach Anspruch 7, **dadurch gekennzeichnet, dass** der Zubehörteil (3) eine Sonde zum Messen des pH-Werts des Inhalts, der sich im Inneren (4) des Behälters (2) befindet, ist.

9. Einheit (1 + 2 + 3) mit Behälter zur biopharmazeutischen Anwendung mit Messung oder Kontrolle eines Parameters einer Flüssigkeit, die sich im Inneren (4) des Behälters (2) befindet, umfassend einen Behälter (2) mit einer Wand (6), die mit einer Öffnung (7) und, starr und dicht an der Wand (6) um die Öffnung (7) befestigt, einem festen und dichten komplementären Verbindungsteil (19) versehen ist, der geeignet ist, mit dem proximalen Endteil (30) zur festen Verbindung des ersten Mittels (15) einer Verbindungsvorrichtung (1) nach einem der Ansprüche 1 bis 6 oder einer Einheit zur Messung oder Kontrolle (1 + 3) nach einem der Ansprüche 7 und 8 zusammenzuwirken, und/oder **dadurch gekennzeichnet, dass** der komplementäre Teil zur festen und dichten Verbindung (19) eine allgemeine röhrenförmige Form mit einer Seitenwand (19a) hat, mit einem proximalen Ende mit Öffnung (20) und einem distalen Ende mit Öffnung (21), und mit einem proximalen Endkragen (23), der geeignet ist, auf feste und dichte Weise mit der Wand (6) des Behälters (2) um seine Öffnung (7) verbunden zu werden.

10. Einheit (1 + 2 + 3) mit Behälter nach Anspruch 9, **dadurch gekennzeichnet, dass** der Behälter (2) entweder starr und wiederverwendbar oder flexibel und zur Einfachverwendung, wie eine so genannte 2D- oder 3D-Tasche, ist, und/oder bei der der Behälter (2) flexibel ist, **dadurch gekennzeichnet, dass** starre Mittel zum äußeren Halten des Behälters (2) vorgesehen sind, die mit einem Durchgang für die Verbindungsvorrichtung (1) oder die Einheit zur Messung oder Kontrolle versehen sind.

11. Verfahren zur Vorverbindung einer Einheit (1 + 3) zur Messung oder Kontrolle eines Parameters nach einem der Ansprüche 7 und 8, im Hinblick auf ihre spätere Montage auf einem Behälter (2), **gekennzeichnet durch** die Aufeinanderfolge der folgenden Schritte:
- Vorhandensein von Mitteln, die geeignet sind, eine Verbindungsvorrichtung (1) nach einem der Ansprüche 1 bis 6 darzustellen,
- Vorhandensein eines Zubehörteils zur Messung oder Kontrolle (3),
- Anordnen des zweiten Mittels (16) der Verbindungsvorrichtung (1) und des Zubehörteils (3) derart, dass das zweite Mittel (16) den Zubehörteil (3) trägt, so dass sein aktiver Teil (9) in dem Raum (18a) angeordnet ist, der von dem zweiten Mittel (16) ausgenommen wird,
- axiales Gleiten des zweiten Mittels (16) in das erste Mittel (15) von seiner distalen Endöffnung (31) in proximale Richtung bis zum geschlossenen distalen Zustand, und/oder **dadurch** gekennzeichnet, dass im geschlossenen distalen Zustand die geschlossene Kammer (18) mit einer Flüssigkeit zur Konservierung und/oder Eichung des Zubehörteils (3) gefüllt ist.

12. Verfahren zur Vorverbindung nach Anspruch 11, **dadurch gekennzeichnet, dass** es ohne Notwendigkeit der Eingrenzung in einem Reinraum durchgeführt wird.

13. Verfahren zur Montage einer Einheit (1 + 2 + 3) mit Behälter nach Anspruch 8, **gekennzeichnet durch** die Aufeinanderfolge der folgenden Schritte:
- Vorhandensein eines Behälters (2) ohne Inhalt, der um eine Öffnung (7) mit einem komplementären Teil zur Verbindung (19) mit einer Verbindungsvorrichtung (1) versehen ist,
- Vorhandensein einer Einheit (1 + 3) zur Messung oder Kontrolle eines Parameters nach Anspruch 7,
- axiales Gleiten der Einheit (1 + 3) zur Messung oder Kontrolle eines Parameters in den komplementären Verbindungsteil (19) von seiner distalen Endöffnung (31) in proximale Richtung bis in den geschlossenen distalen Zustand, und/oder **gekennzeichnet durch** einen späteren Schritt der Sterilisation der Einheit (1 + 2 + 3) mit Behälter.

14. Verfahren für den Einsatz einer Einheit (1 + 3) zur Messung oder Kontrolle eines Parameters, die Teil einer Einheit (1 + 2 + 3) mit montiertem Behälter nach einem der Ansprüche 8 bis 10 ist, **gekennzeichnet durch** die Aufeinanderfolge der folgenden Schritte:
- wenn die Kammer (18) leer ist, wird das zweite Mittel (16) der Verbindungsvorrichtung (1) **durch** axiales Gleiten vom geschlossenen distalen Zustand in den offenen proximalen Zustand in proximale Richtung übergeführt,
- der Zubehörteil (3) wird eingesetzt.

15. Verfahren für den Einsatz nach Anspruch 14, **dadurch gekennzeichnet, dass** es mindestens einen zusätzlichen Vorgang der Konservierung, Regelung, Kontrolle oder Eichung des Zubehörteils (3) umfasst, insbesondere einen anfänglichen Vorgang, bevor der Behälter (2) mit seinem Inhalt gefüllt wird, einen abschließenden Vorgang, nachdem der Behälter (2) seines Inhalts entleert wurde, und gegebenenfalls einen Vorgang, nachdem der Behälter (2) mit seinem Inhalt gefüllt und bevor er seines Inhalts entleert wurde, während die Einheit zur Messung oder Kontrolle eines Parameters und ihre Verbindungsvorrichtung (1) in dem geschlossenen distalen Zustand sind, und die Kammer (18) dicht verschlossen ist.

## Claims

1. Device (1) for the fluid-tight connection to a receptacle (2) of an accessory (3) of which the active proximal part (9) is intended to be placed in communication with the inside (4) of the receptacle (2) by an opening (7) in the receptacle (2), the device (1) having an axis (1 a) and comprising:
• a first means (15) of generally tubular shape with a proximal end opening (30) and a distal end opening (31), arranged to ensure the attachment of the device (1) to the receptacle (2), to define a passageway between the inside (4) and the outside (8) of the receptacle (2), to form a support that guides the axial sliding of a second means (16) between a closed distal state and an open proximal state along an axial stroke C, to participate in defining a chamber (18),
• a second means (16), generally elongated, mounted axially in the first means (15), with an interposed lateral sealing component (39a, 39b), so as to be axially slidable along the stroke C between the closed distal state and the open proximal state, arranged to comprise a transverse head (14) at its proximal end portion which is adapted, in the closed distal state, to close the opening (7) in a fluid-tight manner, to contain a space (18a) having a proximal wall formed by the adjacent head (14), a distal boundary (27), and a side passage opening (48), to comprise at its distal end portion an axially slidable actuating part (17), and to form a support for the accessory (3) so that its active part (9) is positioned within said space (18a), to participate in defining a chamber (18),
• a chamber (18) including said space (18a) and which, in the closed distal state, is closed off laterally by the first means (15) and, where appropriate, is adapted to contain in a fluid-tight manner a liquid for preservation, adjustment, monitoring, or calibration of the accessory (3), and which, in the open proximal state, is freed of the first means (15) and is thus able to be in communication with the inside (4) by the side passage opening (48),
**characterized in that**
the first means (15) comprises a proximal end portion (32) for fixed assembly by sliding, arranged so as to be assembled in a fixed and fluid-tight manner, with an interposed lateral sealing component (22), to a complementary fixed assembly part (19) of the receptacle (2), by means of at least an axial sliding in the proximal direction,
• the transverse head (14) has a peripheral lateral dimension and shape that fit with those of the inner surface of the first means (15), so as to be axially slidable within the first means (15) in the proximal direction, from its distal opening (31) to its proximal end portion (32), the head (14) being fitted with a lateral sealing component (28) such as a head seal,
• the second means (16) is adapted for pre-assembly to the first means (15) by an axial sliding in the proximal direction, the accordingly pre-assembled first means (15) and second means (16) forming a structural whole adapted for assembly to said complementary assembly part (19) by sliding axially in the proximal direction.

2. Connection device (1) according to claim 1, wherein, in the closed distal state, the head (14) is located at or adjacent to the proximal opening (30) of the first means (15), and/or the actuating part (17) is located away from the distal end portion (33) of the first means (15), in the distal direction, by a distance at least equal to the stroke C and/or wherein, in the open proximal state, the head (14) is located away from the proximal opening (30) of the first means (15), in the proximal direction, by a distance at least equal to the stroke C, and/or the actuating part (17) is located at or adjacent to or near the distal end portion (33) of the first means (15), in particular outside the first means (15).

3. Connection device (1) according to any one of claims 1 and 2, wherein the first means (15) comprises, in particular consists of, a single first part (15), and/or the second means (16) comprises, in particular consists of, a single second part (16), with mounted lateral sealing components (39a, 39b) and/or wherein the first means (15) comprises a first part (15) of generally tubular shape extending in the axial direction for a length that is greater than the stroke C, in particular substantially greater than the stroke C, having an outer surface of a peripheral lateral dimension and shape corresponding to those of the inner surface of said complementary assembly part so as to be suitable for mounting axially within said complementary assembly part (19), said first part (15) having a lateral radial outward flange (25) adapted to engage with an edge (21) of said complementary assembly part (19) to prevent axial movement in the proximal direction and/or a sealing component (22) placed between the outer surface of said first part (15) and the inner surface of said complementary assembly part (19), in particular a seal (22) supported by the outer surface of said first part (15).

4. Connection device (1) according to any one of claims 1 to 3, wherein
the second means (16) comprises a second part (16) of generally tubular shape extending in the axial direction for a length that is greater than that of the first means (15), in particular at least equal to that of the first means (15) plus the stroke C, more particularly for a length close to that of the first means (15) plus the stroke C and/or a second part (16) equipped at its distal end portion (26) with a lateral radial outward flange (17) adapted to engage with the edge of the distal end portion (33) of the first means (15) to prevent axial movement in the proximal direction, forming the actuating part (17) where appropriate and/or
the second means (16) is mounted axially within the first means (15) with one or more interposed lateral sealing components (39a, 39b) in the form of one or more seals supported by the outer surface of the second means (16), and/or wherein a distal sealing component (39a) and a proximal sealing component (39b) spaced apart from one another in the axial direction, and/or wherein the distance in the axial direction between the distal sealing component (39a) and the proximal sealing component (39b) is greater, in particular slightly greater, than the distance in the axial direction between the proximal sealing component (39b) and the head sealing component (28), and/or wherein the distance in the axial direction between the proximal sealing component (39b) and the head sealing component (28) is close to or equal to the stroke C.

5. Connection device (1) according to any one of claims 1 to 4, wherein a channel (11) for liquid is arranged in the second means (16), such as a bore hole, extending axially, operatively associated with the accessory (3) in particular for preservation, adjustment, monitoring, or calibration, having a proximal end opening (12) into said space (18a), respectively into said chamber (18), and a distal end opening (13) which opens towards the distal end portion (26) of the second means (16), arranged so that the channel is able to be extended distally beyond the second means (16), by an external connection component (5), such as comprising at least one tube and/or comprising at least two channels (11) for respectively feeding and draining.

6. Connection device (1) according to any one of claims 1 to 5, wherein the first means (15) and the second means (16) are pre-assembled to form a structural whole in the closed distal state and/or wherein the accessory (3) is attached from the very start to the second means (16), in particular by overmolding the distal portion (10) of the accessory (3), and/or the closed chamber (18) is filled with a calibration liquid for the accessory (3).

7. Assembly (1 + 3) for the measurement or monitoring of a parameter of a liquid contained inside (4) a receptacle (2) by means of a measurement or monitoring accessory (3) of which the active proximal part (9) is intended to be placed in communication with the inside (4) of the receptacle (2) by an opening (7) in the receptacle (2), said assembly (1 + 3) for measurement or monitoring comprising said accessory (3), a device (1) for the connection of said accessory (3) to the receptacle (2) according to any one of claims 1 to 6, and, where appropriate, a calibration liquid for the accessory (3) filling the closed chamber (18) of said connection device (1) in the closed distal state, said assembly for measurement or monitoring (1 + 3) being ready for mounting onto a receptacle (2), and/or the connection device (1) is of the type in which one or more channels (11) are provided in the second means (16), **characterized by** the fact that it also comprises, associated with the channel (11) or with each of the channels (11) by a fixed and communicating connection, the external connection component (5), such as comprising at least one tube, and/or either comprises one or more components (5a) for connection, stopping flow, allowing flow, filtration, measurement, containment for the supply or collection of the liquid intended to pass through said external connection component and said associated channel, or comprises one or more sampling components (5a) able to collect a sample of the receptacle (2) contents trapped in the chamber after the connection device (1) transitions from the open state to the closed state.

8. Assembly (1 + 3) for measurement or monitoring according to claim 7, wherein the accessory (3) is a probe for measuring the pH of the contents contained within the inside (4) of the receptacle (2).

9. Assembly (1 + 2 + 3) with receptacle for biopharmaceutical applications with measurement or monitoring of a parameter of a liquid contained inside (4) the receptacle (2), comprising: a receptacle (2), having a wall (6) provided with an opening (7); and, attached in a rigid and fluid-tight manner to said wall (6) around the opening (7), a complementary assembly part (19) for fixed and fluid-tight assembly, adapted to cooperate with the proximal end portion (30) for the fixed assembly of the first means (15) of a connection device (1) according to any one of claims 1 to 6 or of an assembly for measurement or monitoring (1 + 3) according to any one of claims 7 et 8, and/or wherein the complementary assembly part (19) for fixed and fluid-tight assembly has a generally tubular shape having a side wall (19a) with a proximal end having an opening (20) and a distal end having an opening (21), and a flange (23) at the proximal end adapted for securing in a fixed and fluid-tight manner to the wall (6) of the receptacle (2) around its opening (7).

10. Assembly (1 + 2 + 3) with receptacle according to claim 9, wherein the receptacle (2) is either rigid and reusable or is flexible and disposable, such as a bag referred to as a 2D or 3D bag, and/or wherein the receptacle (2) is flexible, **characterized by** rigid external support means being provided for the receptacle (2), equipped with a passage for the connection device (1) or for the assembly for measurement or monitoring.

11. Method for the pre-assembly of an assembly (1 + 3) for measurement or monitoring of a parameter according to any one of claims 7 and 8, for its subsequent mounting onto a receptacle (2), **characterized by** the following successive steps:
• means appropriate for constituting a connection device (1) according to any one of claims 1 to 6 are provided,
• a measurement or monitoring accessory (3) is provided,
• the second means (16) of the connection device (1) and the accessory (3) are arranged so the second means (16) supports the accessory (3) such that its active part (9) is located within said space (18a) formed by the second means (16),
• the second means (16) is slid axially in the proximal direction within the first means (15) from its distal end opening (31), to the closed distal state, and/or wherein, in the closed distal state, the closed chamber (18) is filled with a liquid for the preservation and/or calibration of the accessory (3).

12. Pre-assembly method according to claim 11, **characterized by** its being carried out with no need for containment within a clean room.

13. Method for mounting an assembly (1 + 2 + 3) with receptacle according to claim 8, **characterized by** the following successive steps:
• a receptacle (2) empty of content is provided that is equipped, around an opening (7) of a complementary assembly part (19), with a connection device (1),
• an assembly (1 + 3) for measurement or monitoring of a parameter according to claim 7 is provided,
• the assembly (1 + 3) for measurement or monitoring of a parameter is slid axially in the proximal direction within said complementary assembly part (19) from its distal end opening (31), to the closed distal state, and/or comprising a subsequent step of sterilizing the assembly (1 + 2 + 3) with receptacle.

14. Method for utilizing an assembly (1 + 3) for measurement or monitoring of a parameter as part of an assembly (1 + 2 + 3) with receptacle, mounted according to any one of claims 8 to 10, **characterized by** the following successive steps:
• as the chamber (18) is empty, the second means (16) of the connection device (1) is slid axially in the proximal direction, from the closed distal state to the open proximal state,
• the accessory (3) is utilized.

15. Utilization method according to claim 14, comprising at least one additional operation of preservation, adjustment, monitoring, or calibration of the accessory (3), in particular an initial operation before the receptacle (2) is filled with its contents, a final operation after the receptacle (2) is emptied of its contents, and, where appropriate, an operation after the receptacle (2) is filled with its contents and before it is emptied of its contents, while the assembly for measurement or monitoring of a parameter and its connection device (1) are in the closed distal state and the chamber (18) is closed in a fluid-tight seal.
